# FASCICULE DE BREVET EUROPEEN

(11) **EP 0 949 241 B1**
(45) Date de publication et mention de la délivrance du brevet: **10.12.2003**
(21) Numéro de dépôt: 99105754.8
(22) Date de dépôt: 22.03.1999
(51) Int. Cl.: C07C 209/68, C07C 249/02, C07B 35/08

(54) **Isomérisation stéréospécifique d'amines allyliques à l'aide de ligands phosphorés chiraux**
Stereospezifische Isomerisierung von Allylaminen unter Verwendung von Chiralen Phospho-Liganden
Stereospecific isomerisation of allylic amines using chiral phosphorus ligands

(30) Priorité: 07.04.1998 CH 82298
(43) Date de publication de la demande: 13.10.1999
(73) Titulaire: FIRMENICH SA, 1211 Genève 8 (CH)
(72) Inventeur: Chapuis, Christian, 1295 Mies (CH); Barthe, Michel, 1284 Chancy (CH)
(74) Mandataire: Salvaterra-Garcia, Maria de Lurdes, Dr.

(56) Documents cités:
- EP-A- 0 068 506
- EP-A- 0 156 607
- EP-A- 0 170 470
- EP-A- 0 729 969
- US-A- 4 861 890
- V ENEV ET AL: "A bis-steroidal phosphine as new chiral hydrogenation ligand" JOURNAL OF ORGANIC CHEMISTRY., vol. 62, no. 21, 1997, pages 7092-7093, XP002172611 AMERICAN CHEMICAL SOCIETY. EASTON., US ISSN: 0022-3263

## Description

La présente invention a trait au domaine de la synthèse organique catalytique. Plus particulièrement, elle concerne un procédé d'isomérisation stéréospécifique d'amines allyliques, telles que définies par la formule (I) ci-dessous, en utilisant des complexes de certains métaux de transition qui portent en tant que ligand des composés phosphorés, étant soit des ferrocènes plans-chiraux, connus sous le nom de JOSIPHOS ® et de dérivés de celui-ci, soit des ligands biphosphorés dérivés des stéroïdes.

On connaît, depuis plusieurs années déjà, des réactions d'isomérisation d'amines allyliques, prochirales ou non, à l'aide de complexes de rhodium, iridium ou ruthénium tels que, par exemple, ceux représentés par les formules [Rh(P-P)∗diène]⁺X⁻, [Rh(P-P)∗₂]⁺X⁻, [Ru(CH₃COO)₂(P-P)∗] ou [RuY₂(P-P)∗], dans lesquelles (P-P)∗ est un ligand phosphoré bidenté chiral, "diène" représente une dioléfine telle que le cyclooctadiène ou norbomadiène, X- est un anion tel qu'un halogénure, BF⁻₄, PF⁻₆ ou ClO⁻₄, et Y est un halogénure. La réaction d'isomérisation mène aux énamines ou imines correspondantes qui sont ensuite hydrolysées pour obtenir des aldéhydes chiraux, par exemple le citronellal, le méthoxycitronellal ou l'hydroxycitronellal. Ces composés sont des matières hautement appréciées en parfumerie.

Ces procédés connus font l'objet de plusieurs publications. A ce titre, il convient de citer les brevets EP-B-068 506, 135 392 et 156 607 (titulaire : Takasago Perfumery Co.) et JP 61-19203 du même titulaire, ainsi que le brevet EP-B-398 132 (titulaire : Hoffmann-La Roche AG) et la demande de brevet EP 643 065 (demanderesse : Bayer AG). Tous les procédés décrits dans ces références utilisent des catalyseurs portant en tant que ligand des phosphines bidentées chirales qui sont basées sur des systèmes biphényles ou binapthyles. Le ligand le plus connu pour cette réaction d'isomérisation, et qui est aussi utilisé dans d'autres procédés catalytiques, est le BINAP, représenté par la formule suivante (IV)

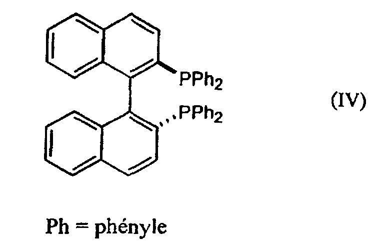

Dans l'ensemble de l'art antérieur sur l'isomérisation, notamment stéréospécifique, d'allylamines, nous n'avons pu trouver aucun exemple d'utilisation d'un autre type de ligand que celui décrit ci-dessus, qui est un ligand phosphoré bidenté et basé sur des biaryles atropisomères.

Ainsi, c'est de façon surprenante que nous avons réussi à développer un autre système catalytique qui fait recours à des ligands phosphorés chiraux d'une structure différente, dérivés du ferrocène, et dont la chiralité n'est pas basée sur une isomérie atropique. Dans un autre mode d'execution de la présente invention, on utilise des phosphines chirales dériveés des stéroïdes. Tous ces ligands se revèlent très utiles pour l'isomérisation susmentionnée d'allylamines prochirales.

L'objet de la présente invention est donc un procédé pour isomériser de façon stéréospécifique des systèmes allyliques prochiraux représentés par la formule 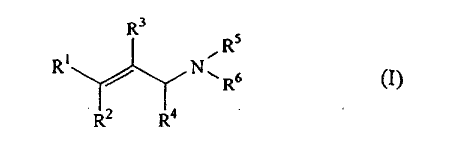 dans laquelle R¹ ≠ R², et chacun représente un groupe alkyle ou alkényle, contenant de 1 à 12 atomes de carbone, ou un groupe aryle, éventuellement substitués par un groupe hydroxy, R³ et R⁴ représentent indépendamment l'un de l'autre l'hydrogène, un groupe alkyle ou alkényle de C₁ à C₁₂, ou un groupe aryle, R⁵ est hydrogène ou un groupe alkyle ou cycloalkyle, contenant de 1 à 8 atomes de carbone, R⁶ est un groupe alkyle ou cycloalkyle, contenant de 1 à 8 atomes de carbone, ou R⁵ et R⁶ sont pris ensemble avec l'azote pour former un cycle ayant 5 ou 6 atomes, ou encore un cycle ayant 6 atomes et contenant l'oxygène,
en des énamines représentées par la formule 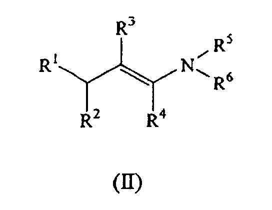 dans laquelle les symboles R¹-R⁶ ont les significations données ci-dessus, ou en des imines représentées par la formule 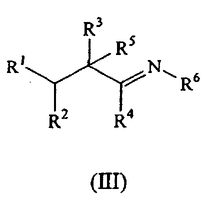 dans laquelle les symboles R¹-R⁴ et R⁶ ont les significations données ci-dessus et R⁵ est l'hydrogène, à l'aide de catalyseurs de Rh, Ir ou Ru portant au moins un ligand phosphoré chiral, le procédé étant caractérisé en ce que le ligand phosphoré dans le catalyseur de Rh, Ir ou Ru est un ligand représenté par la formule (V) 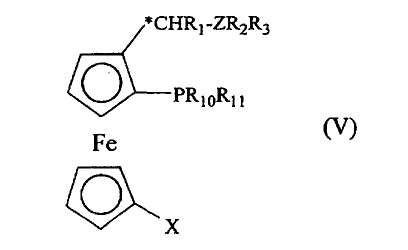 dans laquelle l'atome de carbone marqué par l'astérisque représente un atome de carbone asymétrique, X est un atome d'hydrogène ou un groupe pontant lié à un polymère et représenté par la formule générale (A)ₙ-B-P- dans laquelle
n = 0 ou 1,
A est un groupe intercalaire ("spacer") approprié contenant au moins un atome choisi parmi l'oxygène, le soufre, le silicium, l'azote et le carbone,
B est un groupe liant le polymère P et le reste cyclopentadienyle, et
P est un polymère organique ou inorganique.

A. Z représente un atome de phosphore ; R₁ représente un groupe alkyle de C₁ à C₈, un groupe phényle ou un groupe phényle substitué de 1 à 3 groupes alkyle ou alkoxy de C₁ à C₄ ; R₂, R₃, R₁₀ et R₁₁ signifient, indépendamment l'un de l'autre, un groupe alkyle de C₁ à C₁₂, un groupe cycloalkyle de C₅ à C₁₂, eventuellement substitué par un groupe alkyle ou alkoxy de C₁ à C₄, ou un groupe phényle eventuellement substitué de 1 à 3 groupes sélectionnés parmi des groupes alkyle ou alkoxy de C₁-C₄, -SiR₄R₅R₆, halogène, -SO₃M, -CO₂M, -PO₃M, -NR₇R₈ ou -[⁺NR₇R₈R₉]Y⁻ ou R₂, R₃, R₁₀ et R₁₁ représentent indépendamment l'un de l'autre un reste de formule 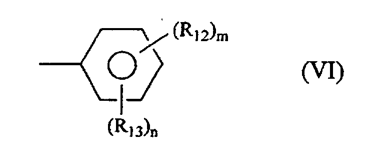 dans laquelle
   R₁₂ représente un groupe alkyle de C₁ à C₅ entièrement ou partiellement substitué de fluor ;
   R₁₃ représente un groupe alkyle ou alkoxy de C₁-C₄, -SiR₄R₅R₆, halogène, -SO₃M, -CO₂M, -PO₃M, -NR₇R₈ ou -[⁺NR₇R₈R₉]Y⁻, et
   m étant un nombre de 1 à 3, n étant 0 ou un nombre de 1 à 4 et la somme de m + n étant 1 à 5 ;
   R₄, R₅ et R₆ signifiant indépendamment l'un de l'autre un groupe alkyle de C₁-C₁₂ ou un groupe phényle ; R₇ et R₈, pris séparément, signifiant chacun un atome d'hydrogène, un groupe alkyle de C₁-C₁₂ ou un groupe phényle, ou pris ensemble représentant un groupe tétraméthylène, un pentaméthylène ou un 3-oxa-1,5-pentylène ; et R₉ représente l'hydrogène ou un groupe alkyle de C₁ à C₄ ;
   M signifiant H ou un métal alkalin, et Y⁻ signifiant l'anion d'un acide monobasique ; ou
B. Z représente l'azote, R₁ représente un groupe alkyle de C₁ à C₈, un groupe phényle ou un groupe phényle substitué de 1 à 3 groupes alkyle ou alkoxy de C₁ à C₄, R₂ et R₃ représentent indépendamment l'un de l'autre un groupe alkyle de C₁ à C₁₂, un groupe cycloalkyle de C₅ à C₁₂, ou un groupe phényle pouvant être substitué de 1 à 3 groupes alkyle de C₁ à C₄ ; ou R₂ et R₃ sont pris avec l'azote pour former un cycle ayant de 5 à 12 atomes et pouvant comporter, de surcroît, d'autres atomes d'oxygène ou d'azote ; et R₁₀ et R₁₁ représentent chacun un groupe phényle.

Des ligands préférés de l'invention sont décrits par la formule générale 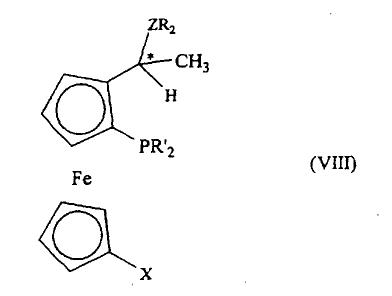 dans laquelle l'atome de carbone marqué de l'astérisque représente un atome de carbone asymétrique, X est l'hydrogène ou un groupe pontant lié à un polymère tel que défini ci-dessus et Z est un atome de phosphore, et R et R' sont, indépendamment l'un de l'autre, un groupe alkyle de C₁ à C₄, linéaire ou ramifié, un groupe cyclohexyle, ou un groupe phényle non substitué ou substitué de 1 à 3 groupes alkyle de C₁ à C₄, ces derniers pouvant être partiellement ou entièrement fluorés ;
ou dans laquelle Z est un atome d'azote, et R et R' sont, indépendamment l'un de l'autre, un groupe alkyle de C₁ à C₄, linéaire ou ramifié, un groupe cyclohexyle, ou un groupe phényle non substitué ou substitué de 1 à 3 groupes alkyle de C₁ à C₄.

Parmi les ligands de formule (VIII) comme définie ci-dessus, on préfère ceux dans laquelle
Z = P
et R = tert-butyle, R' = phényle ; ou R = R' = cyclohexyle ; ou R ≠ R' = cyclohexyle, phényle ; ou R = tert-butyle, R' = p-CF₃-phényle ; ou R = cyclohexyle, R' = p-CF₃-phényle ; ou R = cyclohexyle, R' = p-tolyle ;
ou ZR₂ représente le groupe N(CH₃)₂, et
R' représente un groupe phényle.

Dans un mode d'exécution de la présente invention, les ligands des formules (V) et (VIII) sont non immobilisés, avec X représentant l'hydrogène. Une description détaillée de ce type de ligand et de sa préparation se trouve dans les demandes de brevet EP-A-564 406, 612 758 et 646 590. Les ligands décrits dans ces documents se prètent à une utilisation dans la présente invention. Le contenu de ces documents concernant lesdits ligands et leur préparation est une partie intégrale de la présente demande et est incorporé ici par référence.

Dans un autre mode d'exécution de la présente invention, le ligand utilisé est un ligand immobilisé. De tels ligands immobilisés sont porteurs d'un groupe X lié à un polymère comme mentionné dans les formules (V) et (VIII) ci-dessus, ledit groupe X étant défini par la formule générale (IX) suivante. Pour des raisons de simplicité, on ne donnera ici que la formule la plus générale des ligands immobilisés correspondant aux ligands de formule (V), sans pour autant répéter cette définition pour les ligands qui sont désignés en haut comme étant préférés.

Les ligands immobilisés et utilisés selon la présente invention sont ceux de formule générale 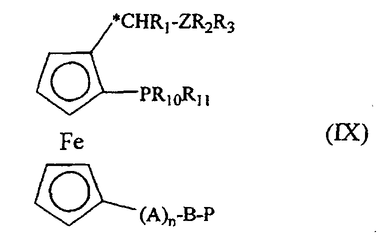 dans laquelle les symboles Z et R₁, R₂, R₃, R₁₀ et R₁₁ ont les significations données ci-dessus et
n = 0 ou 1,
A est un groupe intercalaire ("spacer") approprié contenant au moins un atome choisi parmi l'oxygène, le soufre, le silicium, l'azote et le carbone,
B est un groupe liant le polymère P et le reste cyclopentadienyle, et
P est un polymère organique ou inorganique.

Dans un mode d'exécution préféré qui se réfère à l'utilisation des ligands immobilisés de la formule (IX),
a) n = 0 et
   B est un radical de formule 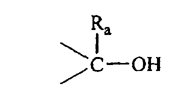
   dans laquelle Rₐ est un atome d'hydrogène ou un groupe alkyle de C₁ à C₁₂ ; ou

b) n = 1 et
   A est un groupe -[Si(R₁₄R₁₅)]ₘR₁₆-, dans lequel m est 0 ou 1, les restes R₁₄ et R₁₅ peuvent être identiques ou différents et représentent indépendamment l'un de l'autre un groupe alkyle de C₁ à C₁₂, un groupe cycloalkyle de C₃ à C₇, un groupe benzyle, un groupe phényle, ou, lorsque pris ensemble, représentent un groupe alkylène de C₅ à C₁₂, et R₁₆ représentant un groupe phénylène ou un groupe alkylène de C₁ à C₁₂, non substitué ou substitué par un ou plusieurs atomes d'halogène ou groupes OH, et
   B est un groupe
      -N(R₁₇)-C(O)-N(H)-(CH₂)ₒ-Si(R₁₈)₃₋ₚ-(O)ₚ- ou un groupe
      -O-C(O)-N(H)(CH₂)ₒ-Si(R₁₈)₃₋ₚ-(O)ₚ- dans lesquels R₁₇ représente un atome d'hydrogène ou un groupe alkyle de C₁ à C₁₂, o est un nombre entier de 1 à 12, R₁₈ est un groupe alkyle de C₁ à C₄ ou un groupe alkoxy de C₁ à C₁₂, et p est un nombre entier de 1 à 3, et
   P est la silice ; ou
c) n = 1 et
   A est un groupe -[Si(R₁₄R₁₅)]ₘR₁₆-, dans lequel m est 0 ou 1, les restes R₁₄ et R₁₅ peuvent être identiques ou différents et représentent indépendamment l'un de l'autre un groupe alkyle de C₁ à C₁₂, un groupe cycloalkyle de C₃ à C₇, un groupe benzyle, un groupe phényle, ou, lorsque pris ensemble, représentent un groupe alkylène de C₅ à C₁₂, et R₁₆ représentant un groupe phénylène ou un groupe alkylène de C₁ à C₁₂, non substitué ou substitué par un ou plusieurs atomes d'halogène ou groupes OH, et
   B est choisi parmi
      i) des groupes -N(R₁₇)-C(O)-, N(R₁₇)-C(O)-N(H)-R₁₉-N(H)-C(O)- ou - O-C(O)-N(H)-R₁₉-N(H)-C(O)- dans lesquels R₁₇ a le sens indiqué ci-dessus, R₁₉ est un groupe pontant dérivé d'un diisocyanate choisi parmi des groupes alkylène de C₂ à C₂₀, substitués ou non substitués, des groupes phénylène, naphtylène et biphénylène, substitués ou non substitués,
      ii) des groupes susceptibles de réagir, par polymérisation ou copolymérisation, avec le monomère qui forme le polymère P, et
      iii) des groupes susceptibles de réagir avec le polymère P ou des groupes fonctionnels présents sur le polymère P, et

   P est un polymère organique, fonctionalisé ou non fonctionalisé.

Par polymère organique, on entend ici, par exemple, des polyoléfines, polyacrylates, polyisoprènes, polybutadiènes, polystyrènes, polyphénylènes, le chlorure de polyvinyle, le chlorure de polyvinylidène, des polyallyle polymères, des polyuréthanes, des polyéthers, des polyesters et des polyamides. Le cas échéant, les polymères peuvent être fonctionalisés. Comme il ressort de ce qui précède, le groupe B peut réagir avec le monomère respectif du polymère P par polymérisation ou copolymérisation, et les ligands immobilisés seront alors obtenus en mélangeant ledit monomère et le ligand non immobilisé porteur d'un groupe approprié B et en soumettant ce mélange à des conditions de polymérisation appropriées qui sont connues de l'homme de métier. Il est également possible d'utiliser des polymères P ayant des groupes fonctionnels qui sont capables de réagir avec des groupes B pour donner les ligands immobilisés tels qu'utilisés dans la présente demande. Ces groupes fonctionnels peuvent être des groupes qui sont présents sur le polymère en tant que tel, ou des groupes introduits par fonctionalisation du polymère. Des polymères appropriés, fonctionalisés ou non fonctionalisés, et des groupes B sont connus de l'homme de métier.

Les ligands de formule (IX) font l'objet des demandes EP-A-729 969 et WO 98/01457. Le contenu de ces demandes citées concernant des polymères appropriés et des groupes liant ces polymères au reste cyclopentadiényle (désignés en haut par A et B) fait partie de la présente demande et est incorporé par référence.

Le ligand phosphoré peut aussi être un ligand représenté par la formule générale 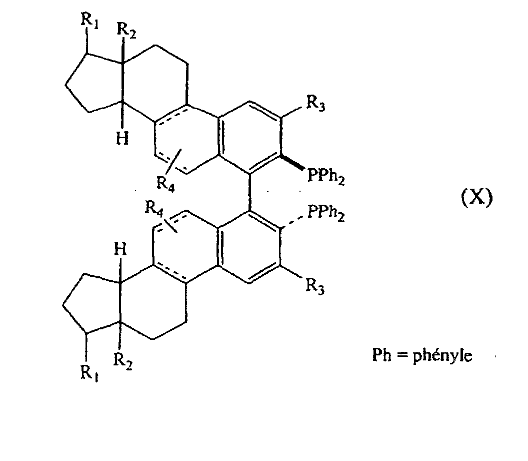 dans laquelle
R₁ représente l'hydrogène, le fluorure, un groupe alkyle ou acyle, ou un groupe de formule XR₅ dans laquelle X représente un atome d'oxygène ou de soufre et R₅ est l'hydrogène ou un groupe alkyle ou aryle ;
R₂ représente l'hydrogène ou un groupe alkyle, la stéréochimie des atomes C-₁₃, C-₁₄ et C-₁₇ pouvant être α ou β ;
R₃ représente l'hydrogène, le fluorure, un groupe alkyl, aryl ou trialkylsilyl ou un groupe de formule XR₆ dans laquelle X a la signification donnée ci-dessus et R₆ représente l'hydrogène ou un groupe alkyle, aryle ou trifluorométhylsulfonyle ;
R₄ est un substituent dans la position 6 ou 7 du stéroïde est représente l'hydrogène, le fluorure, un groupe alkyle ou aryle ou un groupe de formule YR₇ dans laquelle Y représente un atome d'oxygène ou de soufre ou un groupe trialkylsilyle et R₇ représente l'hydrogène ou un groupe alkyle, aryle ou trifluorométhylsulfonyle, et le cycle B du stéroïde est porteur d'aucune ou deux double liaisons.

Dans un mode d'exécution préféré, le ligand représenté par la formule générale (X) ci-dessus est un ligand de formule 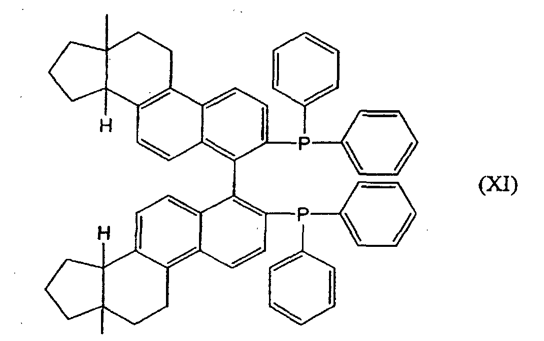 ou 4,4'-bis(diphenylphosphino-13-estra-1,3,5(10),6,8-pentaene).

Les ligands biphosphorés représentés par la formule (IX) sont décrits en détail dans la demande de brevet EP-A-849 274. Le contenu de cette référence concernant les ligands divulgués est une partie intégrale de la présente invention et est incorporé dans la présente demande par référence.

Toutes les références citées ci-dessus mentionnent l'utilisation du type de ligand respectif dans des catalyseurs métalliques utiles pour des réactions d'hydrogénation de substrats variés contenant des doubles liaisons oléfiniques ou carbone-azote. Cependant, il n'y a aucune mention dans ces documents que des catalyseurs métalliques portant ces ligands puissent être utilisés dans d'autres types de réactions catalytiques, notamment celles d'isomérisation des substrats allyliques faisant l'objet de la présente invention.

Il convient de noter qu'il est d'autant plus surprenant de constater une activité catalytique des complexes métalliques portant des ligands de formule (V) ci-dessus dans laquelle Z représente l'azote, puisqu'il s'agit du premier exemple décrit de l'utilisation d'un ligand de telle structure dans la réaction d'isomérisation qui fait l'objet de l'invention. A notre connaissance, tous les ligands utilisés à ce jour étaient des diphosphines bidentées, et on ne trouve nulle part dans l'art antérieur un exemple d'utilisation d'un tel ligand dans des catalyseurs utiles pour l'isomérisation des composés (**I**).

Or, ces ligands azotés permettent d'obtenir des catalyseurs métalliques préférés selon l'invention en raison, d'une part, de la récupération du ligand une fois la réaction complète. Cette récupération peut en effet se faire en précipitant le ligand à l'aide d'un acide approprié. D'autre part, les ligands azotés sont aussi utiles en tant que produits de départ pour la synthèse des diphosphines de formule (V) dans lesquelles Z représente le phosphore, et pour cela ils sont aussi avantageux d'un point de vue économique.

En tant que complexe métallique pouvant servir comme catalyseur pour la réaction d'isomérisation, on utilise des complexes de Rh, Ir et Ru, et de préférence des complexes de Rh.

Le principe de synthèse des complexes actifs de rhodium est connue. Cette synthèse comporte la réaction des phosphines chirales des formules ci-dessus avec un complexe précurseur de Rh(I) approprié. En tant qu'exemple de ce dernier, il convient de citer les complexes de type [Rh(ène)₂Y]₂ ou [Rh diène Y]₂, qui réagissent avec les ligands phosphorés en présence d'un sel d'argent de formule AgX, ou les complexes de type [Rh(diène)₂]X, réagissant aussi avec les ligands phosphorés, en menant aux complexes catalytiquement actifs selon l'invention. Le cas échéant, la synthèse des complexes actifs est exécutée après traitement avec de l'hydrogène, à des pressions pouvant aller jusqu'à 100 bars, de préférence jusqu'à 40 bars. En ce qui concerne les complexes actifs, ceux-ci peuvent être décrits par les formules générales [RhL∗(ène)₂]⁺X⁻, [RhL∗diène]⁺X⁻, [RhL∗]⁺X⁻ ou [RhL∗₂]⁺X⁻. Dans ces formules, L∗ indique un ligand phosphoré chiral comme défini par les formules (V), (IX) ou (X) définies ci-dessus, ène indique une oléfine tel que par exemple l'éthylène, le propylène ou le butène, diène indique un diène tel que par exemple le 1,3-butadiène, le 1,3-pentadiène, le cyclopentadiène, le 1,5-hexadiène, le 1,4-cyclohexadiène, le 1,4- ou le 1,5-heptadiène, le 1,4- ou le 1,5-cyclcoheptadiène, le 1,4-ou le 1,5-octadiène, le 1,4- ou le 1,5-cyclooctadiène, ou le norbomadiène. Les diènes préférés sont le 1,5-cyclooctadiène (qui sera abrégé en "COD" par la suite) et le norbornadiène (qui sera abrégé en "NBD" par la suite). X- indique un anion tel qu'un halogénure, ClO₄⁻, BF₄⁻, B(C₆H₅)₄⁻, PF₆⁻, PCl₆⁻, CH₃COO⁻, CF₃COO⁻, SbF₆⁻, AsF₆⁻, CH₃SO₃⁻, FSO₃⁻ ou CF₃SO₃⁻, et Y indique un anion pontant choisi parmi les halogénures.

Nous avons trouvé que, lorsque le CF₃SO₃⁻, couramment nommé triflate, était utilisé en tant que contre-ion dans le procédé de l'invention, des résultats avantageux étaient obtenus. L'utilisation de cet ion n'a pas seulement souvent mené aux conversions et excès énantiomériques (ee) les plus élevés, par rapport aux ions décrits et utilisés jusqu'à présent dans le type d'isomérisation faisant l'objet de la présente demande, elle a aussi, dans certains modes d'exécution, permis de baisser la proportion du catalyseur par rapport aux substrats. Cet effet sera plus évident à la lecture des exemples présentés plus loin.

Les complexes d'iridium pouvant être utilisés dans le procédé de l'invention sont, d'une façon similaire à ceux de rhodium, synthétisés à partir d'un ligand phosphoré et d'un complexe précurseur approprié. En tant qu'exemple de ces derniers, on cite ici [Ir(COD)Y]₂, IrY₃, [IrY₆]²⁻ dans quelles formules COD signifie le 1,5-cyclooctadiène et Y signifie un anion pontant qui est un halogénure. En ce qui concerne le Ru, on connaît plusieurs complexes de ce métal qui se prêtent à une utilisation dans le procédé de l'invention en tant que complexe précurseur. On ne cite ici, à titre d'exemple non-limitatif, que les espèces [Ru₂(ACOO)₄(H₂O)(diène)₂] (A = groupe alkyle ou aryle non substitué ou halogène), [RuY₂(aryle)]₂ (aryle = groupe aryle comme benzène, toluène, cymène, Y = anion pontant choisi parmi les halogénures).

L'homme de métier connaît un grand nombre d'amines allyliques prochirales pouvant servir comme substrat dans le procédé de l'invention et étant désignés par la formule (I). A ce titre, on fait ici référence aux exemples d'allylamines appropriées citées dans le brevet EP-B-068 506, page 4, lignes 1-7, respectivement les amines allyliques prochirales. Les amines y-citées sont inclues dans cette demande par référence.

Des substrats préférés pour l'isomérisation selon l'invention sont la diéthylnérylamine et la diéthylgéranylamine ainsi que la cyclohexylgéranylamine, la méthylcyclohexylgéranylamine et la (E)- et (Z)-N,N-diéthyl-7-hydroxy-3,7-diméthyl-2-octénylamine [voir K. Tani, Pure Appl. Chem. 1985, (57), 1845 et J. Am. Chem. Soc. 1984 (106) 5208]. Le procédé de l'invention permet d'obtenir, après l'hydrolyse de l'énamine chirale qui se forme lors du procédé, du citronellal optiquement actif.

Le procédé de l'invention permet d'isomériser des amines allyliques avec des conversions pouvant atteindre 100% et des excès énantiomériques de plus de 90%, souvent proche de 100%. Le résultat dépend du complexe et de la phosphine utilisée, ainsi que des conditions de réaction, comme le temps de réaction, la température, la quantité de catalyseur par rapport au substrat, etc. L'homme de l'art est à même d'ajuster ces paramètres de façon à optimiser le rendement de la réaction.

Lorsque le procédé de l'invention est exécuté, le complexe actif peut être synthétisé avant et ensuite ajouté au réacteur, ou être engendré in situ, à partir d'un complexe précurseur, tel que décrit auparavant, et de la phosphine chirale choisie.

La quantité du catalyseur par rapport au substrat peut varier de 0,05 mole% à 20 mole%. De préférence, on utilisera le catalyseur dans une proportion de 0,1 à 5 mole%, par rapport au substrat. Il est possible d'utiliser le catalyseur plusieurs fois, c'est-à-dire une fois la réaction d'isomérisation terminée, le catalyseur est séparé du milieu réactionnel, par exemple par distillation du dernier, et du nouveau substrat est ajouté. Cette opération peut être répétée plusieurs fois sans qu'une désactivation prononcée du catalyseur n'ait lieu.

Lorsque les ligands fixés sur des polymères, par example ceux selon la formule générale (IX), sont utilisés dans la réaction d'isomérisation qui fait l'objet de la présente invention, une séparation du mélange réactionnel du catalyseur est particulièrement facile, car celle-ci peut se faire par simple décantation ou filtration sans qu'une distillation soit nécessaire. Après la séparation du mélange réactionnel, le catalyseur peut être réutilisé dans la réaction d'isomérisation, et on n'observe qu'une très faible désactivation du catalyseur même après plusieurs répétitions du procédé avec le même catalyseur.

Nous avons obtenu les meilleurs résultats en utilisant, en tant que solvant, un alcool, tel que l'éthanol, un ester, tel que l'acétate d'éthyle, ou le tétrahydrofuranne (THF). Ces solvants sont préférés selon l'invention, le THF ayant permis d'obtenir les meilleurs résultats.

La réaction d'isomérisation peut être exécutée à une température comprise entre 0°C et 150°C, de préférence comprise entre 40 et 110°C. La température peut être choisie en fonction du substrat et du solvant utilisé, ceci étant à la portée de l'homme de l'art.

L'invention sera maintenant décrite d'une façon plus détaillée dans la partie expérimentale suivante.

### Partie expérimentale

### A. Préparation générale des composés de départ

Les allylamines de formule (I) sont des produits commerciaux. La diéthylgéranylamine utilisé avait une pureté de 94-99% (déterminée par chromatographie en phase gazeuse ; origine : Dérivés Résiniques et Terpéniques, Castets, France), et la diéthylnérylamine utilisée avait une pureté de 95% et a été préparée comme décrit par K. Takabe, Y. Yamada, T. Katagiri, J. Tanaka dans Org. Synth. 1988, (67), 48.
Les ligands phosphorés ont été fournis par Novartis AG, Bâle, Suisse, Schering AG, Berlin, Allemagne, ou obtenus chez des fournisseurs courants de spécialités chimiques.
Les complexes précurseurs pour la formation des complexes actifs de l'invention peuvent être synthétisés de façon connue de l'homme de l'art, ou comme il est décrit dans les références EP-A-398 132, EP-A-643 065 ou WO 95/21176.

### B. Procédure générale pour le procédé d'isomérisation de diéthylgéranylamine et de diéthylnérylamine

**I.** Complexe précurseur : **[Rh(COD)**_{**2**}**]**^{**+**}**X**^{**-**} (X=anion défini comme plus haut) Dans une boîte à gants, une solution de 0,025 mmole du complexe précurseur, par exemple le [Rh(COD)₂]⁺SO₃CF₃⁻, dans 2,5 ml de THF (0,01 M) a été ajoutée à 0,025 mmole du ligand respectif, dans un ballon à col et muni d'un robinet. La solution a été agitée pendant 1 heure, ensuite une solution de 10 mmole de diéthylgéranylamine ou de diéthylnérylamine dans 10 ml du solvant choisi a été ajoutée. Le ballon a été muni d'un réfrigérant connecté à une ligne Schlenk, purgé à l'argon et porté à reflux. On a maintenu la température constante pendant le temps de réaction indiqué dans le tableau. On a ensuite refroidi la solution à 0°C et ajouté à celle-ci 5 ml d'un mélange 1:4 d'acide acétique/eau. On a laissé monter à température ambiante et ensuite extrait la solution au diéthyléther. L'extrait a été lavé à l'eau (10 ml), NaOH à 15% (2×10 ml) et ensuite à neutralité avec de l'eau, et ensuite séché sur MgSO₄. Après concentration de la solution résultante, le résidu a été distillé dans un four à boules pour fournir du citronellal optiquement actif, avec une conversion et un excès énantiomérique tel qu'indiqué dans les tableaux ci-après.
**II.** Complexe précurseur : **[Rh(COD)Y]**_{**2**} (Y=anion comme défini plus haut) Dans une boîte à gants, une solution de 0,025 mmole du complexe précurseur, par exemple du [Rh(COD)Cl]₂ dans 5 ml de THF (0,005 M) a été rajoutée à 0,055 mmole du ligand respectif, et 0,19 mmole du sel d'argent AgX respectif, dans un ballon. La solution a été agitée pendant 1 heure et ensuite transférée avec une seringue, sous filtration à travers un filtre Acrodisk® dans un ballon à col et muni d'un robinet. On a ensuite procédé comme décrit à la procédure I ci-dessus.
**III.** Complexe précurseur : **[Rh(COD)**_{**2**}**]**^{**+**}**X**^{**-**} (X=anion comme défini plus haut) et traitement à l'hydrogène après l'addition du substrat.
   Dans une boîte à gants, une solution de 0,025 mmole du complexe précurseur, par exemple le [Rh(COD)₂]⁺BF₄⁻ dans 2,5 ml de THF (0,01 M) a été ajoutée à 0,025 mmole du ligand respectif, dans un ballon à col et muni d'un robinet. La solution a été agitée pendant 1 heure, ensuite une solution de 10 mmole de diéthylgéranylamine ou de diéthylnérylamine dans 10 ml du solvant choisi a été ajoutée. Le ballon a été muni d'un réfrigérant connecté à une ligne Schlenk, purgé à l'hydrogène et après à l'argon. On a porté la solution à reflux pendant le temps de réaction approprié. On a ensuite refroidi la solution obtenue, ajouté 5 ml de pentane et filtré la solution ainsi obtenue, à l'aide d'une seringue, à travers un filtre Acrodisk®. On a ensuite travaillé comme décrit à la procédure I ci-dessus.
**IV.** Complexe précurseur : **[Rh(COD)**_{**2**}**]**^{**+**}**X**^{**-**} (X=anion comme défini plus haut) et traitement à l'hydrogène avant l'addition du substrat.
   Dans une boîte à gants, une solution de 0,025 mmole du complexe précurseur, par exemple le [Rh(COD)₂]⁺BF₄⁻ dans 2,5 ml de THF (0,01 M) a été ajoutée à 0,025 mmole du ligand respectif, dans un ballon à col et muni d'un robinet. La solution a été agitée pendant 1 heure. Le ballon a été muni d'un réfrigérant connecté à une ligne Schlenk, purgé à l'hydrogène et après à l'argon. Ensuite une solution de 10 mmole de diéthylgéranylamine ou de diéthylnérylamine dans 10 ml du solvant choisi a été ajoutée. On a porté la solution à reflux pendant le temps de réaction prévu. On a ensuite refroidi la solution obtenue, ajouté 5 ml de pentane et filtré la solution ainsi obtenue, à l'aide d'une seringue, à travers un filtre Acrodisk®. On a ensuite travaillé comme décrit à la procédure I ci-dessus.

### C. Exemples d'utilisation de ligands spécifiques dans l'isomérisation de diéthylgéranylamine et de diéthylnérylamine

### Remarque générale

Dans les tableaux ci-après, les conversions et les excès énantiomériques (ee) se réfèrent toujours au citronellal obtenu après la réaction d'isomérisation et l'hydrolyse de l'énamine ainsi obtenue. L'isolation de l'énamine ainsi que l'hydrolyse de celle-ci se font par des méthodes connues de l'homme de métier qui sont, par exemple, décrites dans le brevet EP-B-068 506 cité ci-dessus. (+/-) indique si le citronellal formé en quantité prépondérante se trouvait sous forme de l'isomère (+) ou (-). Les excès énantiomériques ont été déterminés par chromatographie en phase gazeuse sur une colonne de type Brechbuehler SA, # 27425-025 ayant une longueur de 25 m et un diamètre de 0,25 mm. Sauf indication contraire sous "mode d'exécution", le complexe précurseur et le ligand ont toujours été employés à une concentration de 1 mole% par rapport au substrat ; dans la même colonne, les chiffres I-IV indiquent si on a travaillé selon la variante, I, II, III ou IV comme décrit auparavant sous B.

### Exemple 1

Le ligand utilisé était la (+)-(S)-N,N-diméthyl-1-[(R)-2-(diphénylphosphino)ferrocényl] éthylamine de formule :

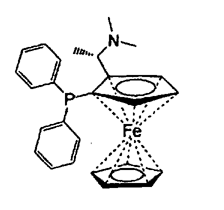

On a procédé comme décrit sous B, en utilisant les complexes précurseurs cités dans le tableau. Les résultats obtenus se trouvent dans le tableau I ci-après :

**Tableau I**

| (substrat : diéthylgéranylamine) | | | | | | | | | | |
|---|---|---|---|---|---|---|---|---|---|---|
| Essai | Mode d'exécution | Complexe précurseur | Temps de complexation | Temps d'hydrogénation | Temps de réaction | Temp. | Conversion | ee | (+/-) | Solvant |
| A | I | [(COD)₂Rh]BF₄ | 01:00 | - | 18:00 | 66°C | 33% | 88% | (+) | THF |
| B | IV | [(COD)₂Rh]BF₄ | 02:30 | 01:00 | 18:00 | 66°C | 79% | 88% | (+) | THF |

### Exemple 2

Le ligand utilisé était la (S)-1-[1-(R)-2-(dicyclohexylphosphino)ferrocényl]éthyldicyclohexyl-phosphine de formule :

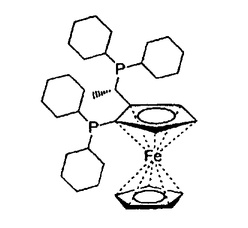

On a procédé comme décrit sous B, en utilisant les complexes précurseurs cités dans le tableau. Les résultats obtenus se trouvent dans le tableau II ci-après :

**Tableau II**

| (substrat : diéthylgéranylamine) | | | | | | | | | | |
|---|---|---|---|---|---|---|---|---|---|---|
| Essai | Mode d'exécution | Complexe précurseur | Temps de complexation | Temps d'hydrogénation | Temps de réaction | Temp. | Conversion | ee | (+/-) | Solvant |
| A | I | [(COD)₂Rh]BF₄ | 01:00 | - | 18:00 | 66°C | 33% | 88% | (+) | THF |
| B | I | [(COD)₂Rh]BF₄ | 01:00 | - | 20:00 | 81°C | 23% | 73% | (+) | CHex |
| C | I | [(COD)₂Rh]BF₄ | 01:00 | - | 20:00 | 77°C | 29% | 79% | (+) | EtOAc |
| D | I | [(COD)₂Rh]SO₃CF₃ | 01:00 | - | 22:00 | 66°C | 81% | 89% | (+) | THF |
| E | I / 0,5mole% de catalyseur | [(COD)₂Rh]SO₃CF₃ | 01:00 | - | 22:00 | 66°C | 72% | 89% | (+) | THF |
| F | I / 0,25mole% de catalyseur | [(COD)₂Rh]SO₃CF₃ | 01:00 | - | 70:00 | 66°C | 33% | 86% | (+) | THF |
| G | III | [(COD)₂Rh]BF₄ | 02:30 | 01:00 | 18:00 | 66°C | 79% | 88% | (+) | THF |
| H | III | [(COD)₂Rh]SO₃CF₃ | 01:00 | 01:30 | 18:00 | 66°C | 99% | 88% | (+) | THF |
| I | III / 2 éq. de ligand | [(COD)₂Rh]SO₃CF₃ | 01:00 | 01:00 | 18:00 | 66°C | 100% | 86% | (+) | THF |
| CHex = cyclohexane | | | | | | | | | | |
| EtOAc = acétate d'éthyle | | | | | | | | | | |

### Exemple 3

Le ligand utilisé était la (R)-1-[1-(S)-2-(diphénylphosphino)ferrocényl]éthyldicyclohexylphosphine de formule :

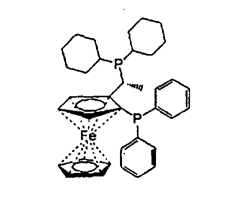

On a procédé comme décrit sous B, en utilisant les complexes précurseurs cités dans le tableau. Les résultats obtenus se trouvent dans les tableaux III ci-après :

**Tableau IIIA**

| (substrat : diéthylgéranylamine) | | | | | | | | | | |
|---|---|---|---|---|---|---|---|---|---|---|
| Essai | Mode d'exécution | Complexe précurseur / sel d'argent | Temps de complexa- | Temps de d'hydrogétion nation | Temps de réaction | Temp. | Conversion | ee | (+/-) | Solvant |
| A | I | [(COD)₂Rh]BF₄ | 01:00 | - | 18:00 | 66°C | 71% | 76% | (-) | THF |
| B | I | [(COD)₂Rh]BF₄ | 01:00 | - | 20:00 | 77°C | 20% | 69% | (-) | EtOAc |
| C | I | [(COD)₂Rh]SO₃CF₃ | 01:00 | - | 21:00 | 66°C | 100% | 74% | (-) | THF |
| D | I | [(COD)₂Rh]BF₄ | 01:00 | - | 21:00 | 78°C | 41% | 67% | (-) | EtOH |
| E | I | [(COD)₂Rh]SO₃CF₃ | 01:00 | - | 21:00 | 78°C | 70% | 64% | (-) | EtOH |
| F | I / 0,5mole% de catalyseur | [(COD)₂Rh]SO₃CF₃ | 01:00 | - | 22:00 | 66°C | 99% | 74% | (-) | THF |
| G | I / 0,2mole% de catalyseur | [(COD)₂Rh]SO₃CF₃ | 01:00 | - | 20:00 | 66°C | 100% | 76% | (-) | THF |
| H | II | [(COD)RhCl]₂-AgClO₄ | 01:00 | - | 20:00 | 66°C | 99% | 72% | (-) | THF |
| I | II | [(COD)RhCl]₂-AgSO₃CF₃ | 01:00 | - | 21:00 | 66°C | 98% | 73% | (-) | THF |
| J | II | [(COD)RhCl]₂-AgPF₆ | 03:00 | - | 19:00 | 66°C | 98% | 79% | (-) | THF |
| K | II | [(COD)RhCl]₂-AgPF₆ | 05:00 | - | 18:00 | 66°C | 98% | 80% | (-) | THF |
| L | II | [(COD)RhCl]₂-AgSbF₆ | 03:00 | - | 19:00 | 66°C | 70% | 77% | (-) | THF |
| M | II | [(COD)RhCl]₂-AgSbF₆ | 05:00 | - | 18:00 | 66°C | 66% | 77% | (-) | THF |
| N | II / 0,5mole% de ligand | [(COD)RhCl]₂-AgPF₆ | 01:00 | - | 20:00 | 66°C | 98% | 85% | (-) | THF |
| O¹⁾ | II / 0,5mole% de ligand | [(COD)RhCl]₂-AgPF₆ | 01:00 | - | 20:00 | 66°C | 99% | 90% | (-) | THF |
| P | IV | [(COD)₂Rh]BF₄ | 01:00 | 00:15 | 18:00 | 66°C | 98% | 74% | (-) | THF |
| Q | II | [(COD)₂Rh]SO₃CF₃ | 01:00 | 00:30 | 22:00 | 66°C | 100% | 67% | (-) | THF |
| R | III / 2 éq. de ligand | [(COD)₂Rh]SO₃CF₃ | 01:00 | 01:30 | 18:00 | 66°C | 97% | 75% | (-) | THF |
| S | III / 2 éq. de ligand | [(COD)RhCl]₂-AgPF₆ | 01:00 | 00:15 | 18:00 | 66°C | 100% | 88% | (-) | THF |

| | | | | | | | | | | |
|---|---|---|---|---|---|---|---|---|---|---|
| 1) solvant séché sur tamis moléculaire EtOH = éthanol EtOAc = acétate d'éthyle | | | | | | | | | | |

**Tableau IIIB**

| (substrat : diéthylnérylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I/0,25mole% de catalyseur | [(COD)₂Rh]SO₃CF₃ | 01:00 | - | 20:00 | 66°C | 100% | 86% | (+) | THF |

### Exemple 4

Le ligand utilisé était la (R)-1-[1-(S)-2-(di-p-tolylphosphino)ferrocényl]éthyldicyclohexylphosphine de formule :

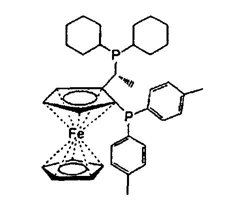

On a procédé comme décrit sous B, en utilisant les complexes précurseurs cités dans le tableau. Les résultats obtenus se trouvent dans le tableau IV ci-après :

**Tableau IV**

| (substrat : diéthylgéranylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I | [(COD)₂Rh]SO₃CF₃ | 01:00 | - | 22:00 | 66°C | 98% | 75% | (-) | THF |
| B | IV | [(COD)RhCl]₂-AgPF₆ | 01:00 | - | 20:00 | 66°C | 100% | 75% | (-) | THF |

### Exemple 5

Le ligand utilisé était la (R)-1-[1-(S)-2-(di-p-trifluorométhylphosphino)ferrocényl] éthyldicyclohexylphosphine de formule :

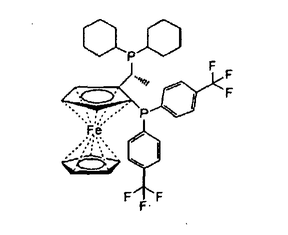

On a procédé comme décrit sous B, en utilisant les complexes précurseurs cités dans le tableau. Les résultats obtenus se trouvent dans le tableau V ci-après :

**Tableau V**

| (substrat : diéthylgéranylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I | [(COD)₂Rh]SO₃CF₃ | 01:00 | - | 22:00 | 66°C | 94% | 79% | (-) | THF |
| B | IV | [(COD)RhCl]₂-AgPF₆ | 01:00 | - | 20:00 | 66°C | 47% | 86% | (-) | THF |

### Exemple 6

Le ligand utilisé était la (R)-1-[1-(S)-2-(diphénylphosphino)ferrocényl]éthyl-di-tert-butylphosphine de formule suivante :

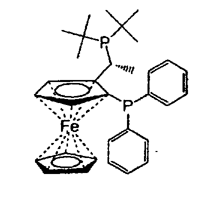

On a procédé comme décrit sous B, en utilisant les complexes précurseurs cités dans le tableau. Les résultats obtenus se trouvent dans les tableaux VI ci-après :

**Tableau VIA**

| (substrat : diéthylgéranylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I | [(COD)₂Rh]BF₄ | 01:00 | - | 18:00 | 66°C | 43% | 90% | (-) | THF |
| B | I | [COD)₂Rh]BF₄ | 01:00 | - | 20:00 | 77°C | 93% | 80% | (-) | EtOAc |
| C | I | [(COD)₂Rh]SO₃CF₃ | 01:00 | - | 21:00 | 66°C | 99% | 83% | (-) | THF |
| D | I / 0,5mole% de catalyseur | [(COD)₂Rh]SO₃CF₃ | 01:00 | - | 22:00 | 66°C | 99% | 89% | (-) | THF |
| E | I / 0,25mole% de catalyseur | [(COD)₂Rh]SO₃CF₃ | 01:00 | - | 70:00 | 66°C | 99% | 92% | (-) | THF |
| F | II | [(COD)RhCl]₂-AgClO₄ | 01:00 | - | 20:00 | 66°C | 98% | 89% | (-) | THF |
| G | II | [(COD)RhCl]₂-AgSO₃CF₃ | 01:00 | - | 21:00 | 66°C | 99% | 72% | (-) | THF |
| H | II | [(NBD)RhCl]₂-AgSO₃CF₃ | 01:00 | - | 21:00 | 66°C | 69% | 91% | (-) | THF |
| I | II | [(NBD)RhCl]₂-AgSO₃CF₃ | 05:00 | - | 18:00 | 66°C | 67% | 92% | (-) | THF |
| J | II | [(NBD)RhCl]₂-AgPF₆ | 01:00 | - | 21:00 | 66°C | 73% | 91% | (-) | THF |
| K | III | [(COD)₂Rh]SO₃CF₃ | 01:00 | 00:30 | 20:00 | 66°C | 100% | 81% | (-) | THF |
| L | III / 2 éq. de ligand | [(COD)₂Rh]SO₃CF₃ | 01:00 | 01:30 | 18:00 | 66°C | 100% | 71% | (-) | THF |
| EtOAc = acétate d'éthyle | | | | | | | | | | |

**Tableau VIB**

| (substrat : diéthylnérylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I / 0,25mole% de catalyseur | [(COD)₂Rh]SO₃CF₃ | 01:00 | - | 20:00 | 66°C | 100% | 97% | (+) | THF |

### Exemple 7

Le ligand utilisé était la (R)-1-[1-(S)-2-(di-p-trifluorométhylphosphino)ferrocényl]éthyl-di-tert-butylphosphine de formule :

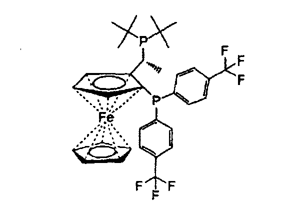

On a procédé comme décrit sous B, en utilisant les complexes précurseurs cités dans le tableau Les résultats obtenus se trouvent dans le tableau VII ci-après :

**Tableau VII**

| (substrat : diéthylgéranylamine) | | | | | | | | | | |
|---|---|---|---|---|---|---|---|---|---|---|
| Essai | Mode d'exécution | Complexe précurseur/sel d'argent | Temps de complexation | Temps d'hydrogénation | Temps de réaction | Temp. | Conversion | ee | (+/-) | Solvant |
| A | I | [(COD)₂Rh]SO₃CF₃ | 01:00 | - | 20:00 | 66°C | 100% | 89% | (-) | THF |
| B | II | [(COD)RhCl]₂-AgPF₆ | 01:00 | - | 20:00 | 66°C | 42% | 90% | (-) | THF |

### Exemple 8

Le ligand utilisé était la (R)-1-[1-(S)-2-(dicyclohexylphosphino)ferrocényl]éthyldiphénylphosphine de formule :

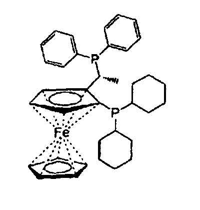

On a procédé comme décrit sous B, en utilisant les complexes précurseurs cités dans le tableau. Les résultats obtenus se trouvent dans les tableaux VIII ci-après :

**Tableau VIIIA**

| (substrat : diéthylgéranylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I | [(COD)₂Rh]BF₄ | 01:00 | - | 18:00 | 66°C | 98% | 79% | (-) | THF |
| B | I | [(COD)₂Rh]SO₃CF₃ | 01:00 | - | 22:00 | 66°C | 100% | 90% | (-) | THF |
| C | II | [(COD)RhCl]₂-AgPF₆ | 01:00 | - | 22:00 | 66°C | 98% | 90% | (-) | THF |
| D | III | [(COD)₂Rh]BF₄ | 02:30 | 01:00 | 18:00 | 66°C | 100% | 72% | (-) | THF |

**Tableau VIIIB**

| (substrat : diéthylnérylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I/0,25mole% de catalyseur | [(COD)₂Rh]SO₃CF₃ | 1:00 | - | 20:00 | 66°C | 100% | 93% | (+) | THF |

### Exemple 9

Le ligand utilisé était la (R)-1-[1-(S)-2-(diphénylphosphino)ferrocényl]éthyldicyclohexylphosphine fixée sur silice de formule :

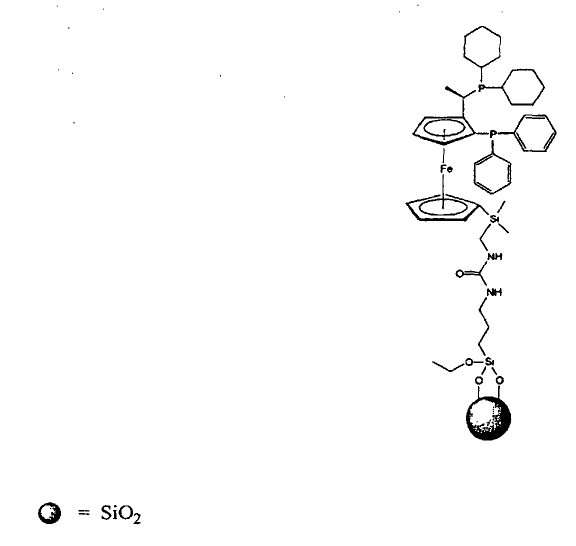

**Tableau IX**

| (substrat : diéthylgéranylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I | [(COD)₂Rh]SO₃CF₃ | 1:00 | - | 19:00 | 66°C | 81% | 83% | (-) | THF |

### Exemple 10

Le ligand utilisé était la (R)-1-[1-(S)-2-(diphénylphosphino)ferrocényl]éthyl-di-tert-butyl-phosphine fixée sur silice de formule

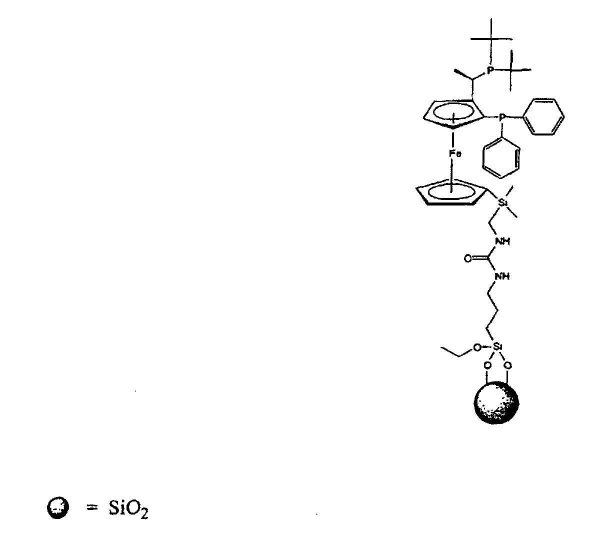

**Tableau X**

| (substrat : diéthylgéranylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I | [(COD)₂Rh]SO₃CF₃ | 1:00 | - | 19:00 | 66°C | 97% | 86% | (-) | THF |
| B | I/0,25mole% de catalyseur | [(COD)₂Rh]SO₃CF₃ | 1:00 | - | 19:00 | 66°C | 97% | 87% | (-) | THF |

### Exemple 11

Le ligand utilisé était la (R)-1-[1-(S)-2-(diphénylphosphino)ferrocényl]éthyldicyclohexylphosphine fixée sur polystyrène de formule

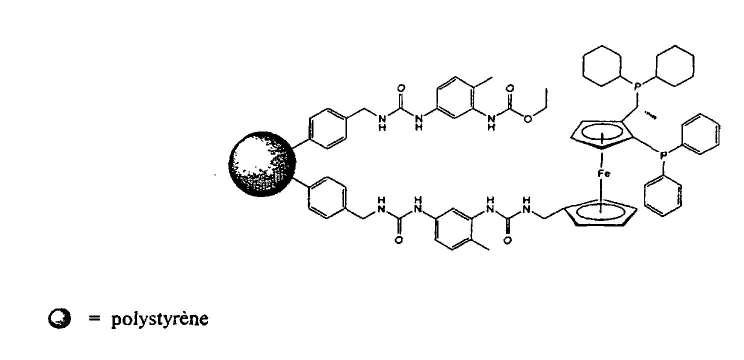

**Tableau XI**

| (substrat diéthylgéranylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I | [(COD)₂Rh]SO₃CF₃ | 1:00 | - | 19:00 | 66°C | 54% | 78% | (+) | THF |

### Exemple 12

Les ligands utilisés étaient les suivants :

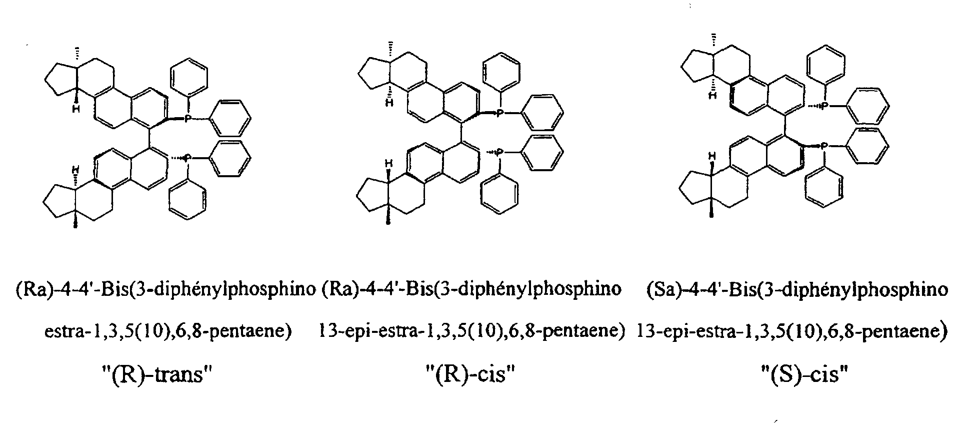

**Tableau XIIA**

| (substrat : diéthylgéranylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | I | [(COD)₂Rh]SO₃CF₃/ "(R)-trans" | 1:00 | - | 20:00 | 66°C | 100% | 87% | (-) | THF |
| B | I | [(COD)₂Rh]SO₃CF₃/ "(R)-cis" | 1:00 | - | 20:00 | 66°C | 100% | 93% | (-) | THF |
| C | I | [(COD)₂Rh]SO₃CF₃/ ''(S)-cis" | 1:00 | - | 6:00 | 66°C | 100% | 89% | (+) | THF |

**Tableau XIIB**

| (substrat diéthylnérylamine) | | | | | | | | | | |
|---|---|---|---|---|---|---|---|---|---|---|
| Essai | Mode d'exécution | Complexe précurseur / sel d'argent | Temps de complexation | Temps d'hydrogénation | Temps de réaction | Temp. | Conversion | ee | (+/-) | Solvant |
| A | 1 | [(COD)₂Rh]SO₃CF₃/ "(R)-cis" | 1:00 | - | 20:00 | 66°C | 100% | 95% | (+) | THF |

## Revendications

1. Procédé d'isomérisation stéréospécifique de systèmes allyliques prochiraux représentés par la formule 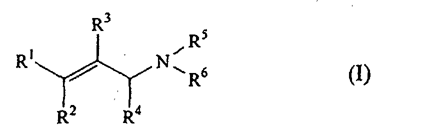 dans laquelle R¹ ≠ R², et chacun représente un groupe alkyle ou alkényle, contenant de 1 à 12 atomes de carbone, ou un groupe aryle, éventuellement substitués par un groupe hydroxy, R³ et R⁴ représentent indépendamment l'un de l'autre l'hydrogène, un groupe alkyle ou alkényle de C₁ à C₁₂, ou un groupe aryle, R⁵ est l'hydrogène ou un groupe alkyle ou cycloalkyle, contenant de 1 à 8 atomes de carbone, R⁶ est un groupe alkyle ou cycloalkyle, contenant de 1 à 8 atomes de carbone, ou R⁵ et R⁶ sont pris ensemble avec l'azote pour former un cycle ayant 5 ou 6 atomes, ou encore un cycle ayant 6 atomes et contenant l'oxygène,
en des énamines représentées par la formule 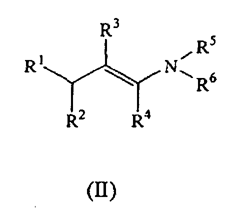 dans laquelle les symboles R¹-R⁶ ont les significations données ci-dessus, ou en des imines représentées par la formule 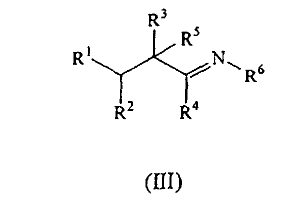 dans laquelle les symboles R¹-R⁴ et R⁶ ont les significations données ci-dessus et R⁵ est l'hydrogène, à l'aide de catalyseurs de Rh, Ir ou Ru portant au moins un ligand phosphoré chiral, le procédé étant **caractérisé en ce que** le ligand phosphoré est un ligand représenté par la formule (V) 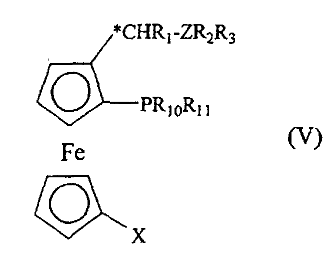 dans laquelle l'atome de carbone marqué de l'astérisque représente un atome de carbone asymétrique, X est un atome d'hydrogène ou un groupe pontant lié à un polymère et représenté par la formule (A)ₙ-B-P dans laquelle
n = 0 ou 1,
A est un groupe intercalaire approprié contenant au moins un atome choisi parmi l'oxygène, le soufre, le silicium, l'azote et le carbone,
B est un groupe liant le polymère P et le reste cyclopentadienyle, et
P est un polymère organique ou inorganique, et
A. Z représente un atome de phosphore ; R₁ représente un groupe alkyle de C₁ à C₈, un groupe phényle ou un groupe phényle substitué de 1 à 3 groupes alkyle ou alkoxy de C₁ à C₄ ; R₂, R₃, R₁₀ et R₁₁ signifient, indépendamment l'un de l'autre, un groupe alkyle de C₁ à C₁₂, un groupe cycloalkyle de C₅ à C₁₂ éventuellement substitué par un groupe alkyle ou alkoxy de C₁ à C₄, ou un groupe phényle éventuellement substitué de 1 à 3 groupes sélectionnés parmi des groupes alkyle ou alkoxy de C₁-C₄, -SiR₄R₅R₆, halogène, -SO₃M, -CO₂M, -PO₃M, -NR₇R₈ ou -[⁺NR₇R₈R₉]Y⁻; ou R₂, R₃, R₁₀ et R₁₁ représentent indépendamment l'un de l'autre un reste de formule 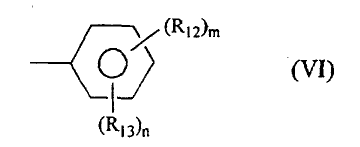
dans laquelle R₁₂ représente un groupe alkyle de C₁ à C₅ entièrement ou partiellement substitué de fluor ;
R₁₃ représente un groupe alkyle ou alkoxy de C₁-C₄, -SiR₄R₅R₆, halogène, -SO₃M, -CO₂M, -PO₃M, -NR₇R₈ ou -[⁺NR₇R₈R₉]Y⁻ ;
m est un nombre de 1 à 3 ; n est 0 ou un nombre de 1 à 4 et la somme de m + n étant 1 à 5;
R₄, R₅ et R₆ ci-dessus signifiant, indépendamment l'un de l'autre, un groupe alkyle de C₁-C₁₂ ou un groupe phényle ; R₇ et R₈, pris séparément, signifiant chacun un atome d'hydrogène, un groupe alkyle de C₁-C₁₂ ou un groupe phényle, ou pris ensemble représentant un groupe tétraméthylène, pentaméthylène ou 3-oxa-1,5-pentylène ; et R₉ représentant l'hydrogène ou un groupe alkyle de C₁ à C₄ ;
M signifiant H ou un métal alkalin, et Y⁻ signifiant l'anion d'un acide monobasique ;
ou
B. Z représente l'azote, R₁ représente un groupe alkyle de C₁ à C₈, un groupe phényle ou un groupe phényle substitué de 1 à 3 groupes alkyle ou alkoxy de C₁ à C₄, R₂ et R₃ représentent, indépendamment l'un de l'autre, un groupe alkyle de C₁ à C₁₂, un groupe cycloalkyle de C₅ à C₁₂ ou un groupe phényle pouvant être substitué de 1 à 3 groupes alkyle de C, à C₄ ; ou R₂ et R₃ sont pris avec l'azote pour former un cycle ayant de 5 à 12 atomes et pouvant comporter, d'autres atomes d'oxygène ou d'azote ; et R₁₀ et R₁₁ représentent chacun un groupe phényle ; ou
le ligand est représenté par la formule générale 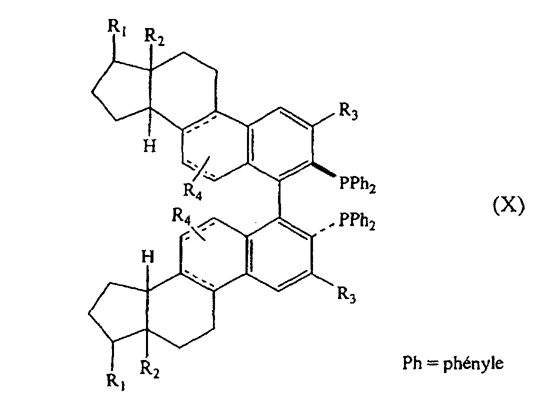 dans laquelle
R₁ représente l'hydrogène, le fluorure, un groupe alkyle ou acyle, ou un groupe de formule XR₅ dans laquelle X représente un atome d'oxygène ou de soufre et R₅ est l'hydrogène ou un groupe alkyle ou aryle ;
R₂ représente l'hydrogène ou un groupe alkyle, la stéréochimie des atomes C-₁₃, C-₁₄ et C-₁₇ pouvant être α ou β ;
R₃ représente l'hydrogène, le fluorure, un groupe alkyle, aryle ou trialkylsilyle ou un groupe de formule XR₆ dans laquelle X a la signification donnée ci-dessus et
R₆ représente l'hydrogène ou un groupe alkyle, aryle ou trifluorométhylsulfonyle ;
R₄ est un substituent dans la position 6 ou 7 du stéroïde et représente l'hydrogène, le fluorure, un groupe alkyle ou aryle ou un groupe de formule YR₇ dans laquelle Y représente un atome d'oxygène ou de soufre ou un groupe trialkylsilyle et R₇ représente l'hydrogène ou un groupe alkyle, aryle ou trifluorométhylsulfonyle ; le cycle B du stéroïde étant saturé ou porteur de deux doubles liaisons.

2. Procédé selon la revendication 1, **caractérisé en ce qu'**on utilise un ligand de formule générale 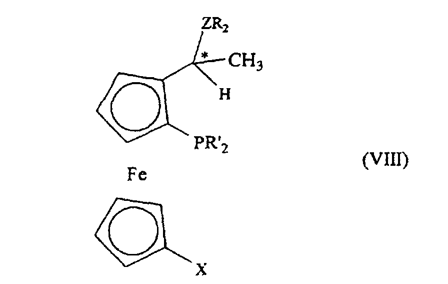 dans laquelle l'atome de carbone marqué de l'astérisque est un atome de carbone asymétrique, X est l'hydrogène ou un groupe pontant lié à un polymère tel que défini à la revendication 1, Z est un atome de phosphore, et R et R' sont, indépendamment l'un de l'autre, un groupe alkyle de C₁ à C₄, linéaire ou ramifié, un groupe cyclohexyle, ou un groupe phényle non substitué ou substitué de 1 à 3 groupes alkyle de C₁ à C₄, ces derniers pouvant être partiellement ou entièrement fluorés ;
ou dans laquelle Z est un atome d'azote, R et R' sont, indépendamment l'un de l'autre, un groupe alkyle de C₁ à C₄, linéaire ou ramifié, un groupe cyclohexyle, ou un groupe phényle non substitué ou substitué de 1 à 3 groupes alkyle de C₁ à C₄.

3. Procédé selon la revendication 1 ou 2, **caractérisé en ce qu'**on utilise un ligand de formule (VIII) dans laquelle Z est un atome de phosphore, R=tert-butyle et R'=phényle ; ou R=R'=cyclohexyle ; ou R≠R'=cyclohexyle et phényle ; ou R=tert-butyle et R'=p-CF₃-phényle ; ou R=cyclohexyle et R'=p-CF₃-phényle ; ou R=cyclohexyle et R'=p-tolyle ; ou Z est un atome d'azote et R=méthyle et R'-phényle.

4. Procédé selon l'une des revendications 1 à 3, **caractérisé en ce que** X est un atome d'hydrogène.

5. Procédé selon l'une des revendications 1 à 3, **caractérisé en ce que** X est un groupe (A)ₙ-B-P dans lequel
a) n = 0 et
B est un radical de formule 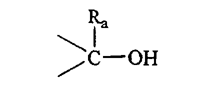
dans laquelle Rₐ est un atome d'hydrogène ou un groupe alkyle de C₁ à C₁₂ ; ou
b) n=1 et
A est un groupe -[Si(R₁₄R₁₅)]ₘR₁₆-, dans lequel m est 0 ou 1, les restes R₁₄ et R₁₅ peuvent être identiques ou différents et représentent indépendamment l'un de l'autre un groupe alkyle de C₁ à C₁₂, un groupe cycloalkyle de C₃ à C₇, un groupe benzyle, un groupe phényle, ou, lorsque pris ensemble, représentent un groupe alkylène de C₅ à C₁₂, et R₁₆ représentant un groupe phénylène ou un groupe alkylène de C₁ à C₁₂, non substitué ou substitué par un ou plusieurs atomes d'halogène ou groupes OH, et
B est un groupe
-N(R₁₇)-C(O)-N(H)-(CH₂)ₒ-Si(R₁₈)₃₋ₚ-(O)ₚ- ou un groupe - O-C(O)-N(H)(CH₂)ₒ-Si(R₁₈)₃₋ₚ-(O)ₚ- dans lesquels R₁₇ représente un atome d'hydrogène ou un groupe alkyle de C₁ à C₁₂, o est un nombre entier de 1 à 12, R₁₈ est un groupe alkyle de C₁ à C₄ ou un groupe alkoxy de C₁ à C₁₂, et p est un nombre entier de 1 à 3, et
P est la silice ; ou
c) n = 1 et
A est un groupe -[Si(R₁₄R₁₅)]ₘR₁₆-, dans lequel m est 0 ou 1, les restes R₁₄ et R₁₅ peuvent être identiques ou différents et représentent indépendamment l'un de l'autre un groupe alkyle de C₁ à C₁₂, un groupe cycloalkyle de C₃ à C₇, un groupe benzyle, un groupe phényle, ou, lorsque pris ensemble, représentent un groupe alkylène de C₅ à C₁₂, et R₁₆ représentant un groupe phénylène ou un groupe alkylène de C₁ à C₁₂, non substitué ou substitué par un ou plusieurs atomes d'halogène ou groupes OH, et
B est choisi parmi
i) des groupes -N(R₁₇)-C(O)-, N(R₁₇)-C(O)-N(H)-R₁₉-N(H)-C(O)- ou - O-C(O)-N(H)-R₁₉-N(H)-C(O)- dans lesquels R₁₇ a le sens indiqué ci-dessus, R₁₉ est un groupe pontant dérivé d'un diisocyanate choisi parmi des groupes alkylène de C₂ à C₂₀, substitués ou non substitués, des groupes phénylène, naphtylène et biphénylène, substitués ou non substitués,
ii) des groupes susceptibles de réagir, par polymérisation ou copolymérisation, avec le monomère qui forme le polymère P, et
iii) des groupes susceptibles de réagir avec le polymère P ou des groupes fonctionnels présents sur le polymère P, et
P est un polymère organique, fonctionalisé ou non fonctionalisé, et choisi parmi des polyolefines, des polyacrylates, polyisoprènes, polybutadiènes, polystyrènes, polyphénylènes, le chlorure de polyvinyle, le chlorure de polyvinylidène, des polyallyle polymères, des polyuréthanes, des polyéthers, des polyesters et des polyamides.

6. Procédé selon la revendication 1, **caractérisé en ce qu'**on utilise un ligand de formule (XI)

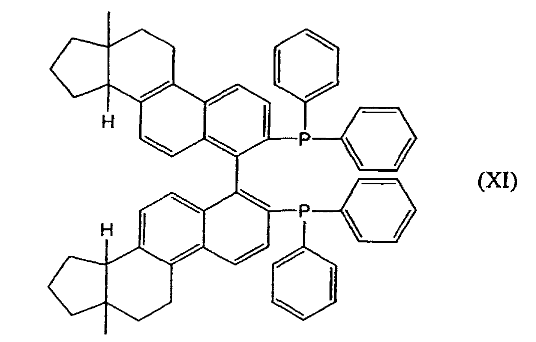

7. Procédé selon l'une des revendications 1 à 6, **caractérisé en ce qu'**on utilise un complexe actif de Rh de formule [RhL∗(ène)₂]⁺X⁻, [RhL∗diène]⁺X⁻ ; [RhL∗]⁺X⁻ ou [RhL∗₂]⁺X⁻, dans lasquelle L∗ représente un ligand phosphoré chiral comme défini dans une des revendications 1 à 7, "ène" représente une oléfine tel qu'éthylène, propylène ou butène, "diène" représente un diène tel que le 1,3-butadiène, le 1,3-pentadiène, le cyclopentadiène, le 1,5-hexadiène, le 1,4-cyclohexadiène, le 1,4- ou le 1,5-heptadiène, le 1,4- ou le 1,5-cyclcoheptadiène, le 1,4- ou le 1,5-octadiène, le 1,4- ou le 1,5-cyclooctadiène, ou le norbornadiène, et X⁻ indique un anion tel qu'un halogénure, ClO₄⁻, BF₄⁻, B(C₆H₅)₄⁻, PF₆⁻, PCl₆⁻, CH₃COO⁻, CF₃COO⁻, SbF₆⁻, AsF₆⁻, CH₃SO₃⁻, FSO₃⁻ ou CF₃SO₃⁻.

8. Procédé selon la revendication 7, **caractérisé en ce que** la diène est la norbornadiène ou la 1,5-cyclooctadiène et l'anion X⁻ est le CF₃SO₃⁻.

9. Procédé selon l'une des revendications 1 à 8, **caractérisé en ce que** l'amine allylique est la géranyldiéthylamine, la néryldiéthylamine, la cyclohexylgéranylamine, la méthylcyclohexylgéranylamine ou la (E)- ou la (Z)-N,N-diéthyl-7-hydroxy-3,7-diméthyl-2-octénylamine.

10. Procédé selon l'une des revendications 1 à 9, **caractérisé en ce que** le complexe actif est utilisé à une concentration comprise entre 0,05 mole% et 20 mole% par rapport au substrat.

11. Procédé selon la revendication 10, **caractérisé en ce que** le complexe actif est utilisé à une concentration comprise entre 0,1 et 5 mole% par rapport au substrat.

12. Procédé selon l'une des revendications 1 à 11, **caractérisé en ce que** la réaction est exécutée dans une gamme de températures de 0°C à 150°C.

13. Procédé selon la revendication 12, **caractérisé en ce que** la réaction est exécutée à des températures comprises entre 40° et 110°C.

14. Procédé selon l'une des revendications 1 à 13, **caractérisé en ce que** le solvant utilisé est le tétrahydrofuranne.

## Claims

1. Process for the stereospecific isomerisation of prochiral allylic systems represented by the formula 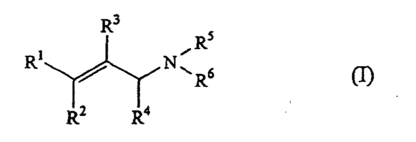 in which R¹ ≠ R², and each represents an alkyl or alkenyl group, containing 1 to 12 carbon atoms, or an aryl group, optionally substituted by a hydroxy group, R³ and R⁴ represent, independently from each other, hydrogen, a C₁ to C₁₂ alkyl or alkenyl group, or an aryl group, R⁵ is hydrogen or an alkyl or cycloalkyl group, containing 1 to 8 carbon atoms, R⁶ is an alkyl or cycloalkyl group, containing 1 to 8 carbon atoms, or R⁵ and R⁶ are taken together with the nitrogen to form a ring having 5 or 6 atoms or a ring having 6 atoms and containing oxygen,
into enamines represented by the formula 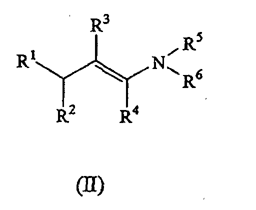 in which the symbols R¹-R⁶ are as defined above, or into imines represented by the formula 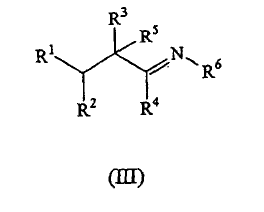 in which the symbols R¹-R⁴ and R⁶ are as defined above and R⁵ is hydrogen, by the means of catalysts of Rh, Ir or Ru carrying at least one chiral phosphine ligand, the process being **characterized in that** the phosphine ligand is a ligand represented by the formula (V) 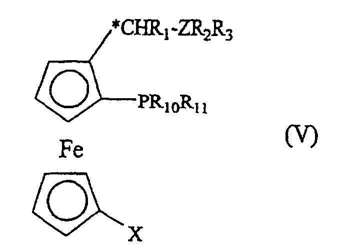 in which the carbon atom marked by an asterisk is an asymmetric carbon atom, X is a hydrogen atom or a bridging group bonded to a polymer and represented by formula (A)ₙ-B-P in which
n is 0 or 1,
A is an appropriate spacer group containing at least one atom selected from the group consisting of oxygen, sulfur, silicon, nitrogen and carbon,
B is a group linking the polymer P and the cyclopentadienyl rest, and
P is an organic or inorganic polymer, and
A. Z stands for a phosphorous atom ; R₁ represents an alkyl group from C₁ to C₈, a phenyl group or a phenyl group substituted by 1 to 3 alkyl or alkoxy groups from C₁ to C₄ ; R₂, R₃, R₁₀ and R₁₁ are, independently from each other, an alkyl group from C₁ to C₁₂, a cycloalkyl group from C₅ to C₁₂, optionally substituted by an alkyl or alkoxy group from C₁ to C₄, or a phenyl group optionally carrying from 1 to 3 substituents selected from the group consisting of alkyl and alkoxy groups from C₁ to C₄, - SiR₄R₅R₆, halides, -SO₃M, -CO₂M, -PO₃M, -NR₇R₈ or -[⁺NR₇R₈R₉]Y⁻ ; or R₂, R₃, R₁₀ and R₁₁ represent, independently from each other, a group of formula 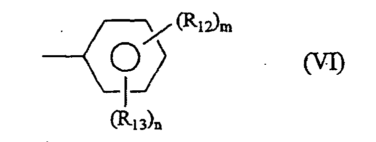 in which
R₁₂ represents an alkyl group from C₁ to C₅, partially or totally fluorinated ;
R₁₃ represents an alkyl or alkoxy groups from C₁ to C₄, -SiR₄R₅R₆, halide, -SO₃M, -CO₂M, -PO₃M, -NR₇R₈ and -[⁺NR₇R₈R₉]Y⁻;
m is an integer from 1 to 3 ; n is 0 or an integer from 1 to 4, and the sum of m + n being from 1 to 5 ;
R₄, R₅ and R₆ hereinabove standing, independently from each other, an alkyl group from C₁ to C₁₂ or a phenyl group; R₇ and R₈, taken separately, represent each hydrogen, an alkyl group from C₁ to C₁₂ or a phenyl group, or, taken together, represent a tetramethylene group, a pentamethylene group or a 3-oxa-1,5-pentylene group; and R₉ is hydrogen or an alkyl group from C₁ to C₄ ;
M standing for H or an alkaline metal, and Y⁻ standing for the anion of a monobasic acid ;
or
B. Z represents a nitrogen, R₁ represents an alkyl group from C₁ to C₈, a phenyl group or a phenyl group substituted by 1 to 3 alkyl or alkoxy groups from C₁ to C₄, R₂ and R₃ represent, independently from each other, an alkyl group from C₁ to C₁₂, a cycloalkyl group from C₅ to C₁₂ or a phenyl group optionally substituted by 1 to 3 alkyl groups from C₁ to C₄ ; or R₂ and R₃ are taken together with the nitrogen to form a ring having from 5 to 12 atoms, optionally comprising further nitrogen or oxygen atoms; and R₁₀ and R₁₁ represent each a phenyl group ; or
the ligand is represented by the general formula 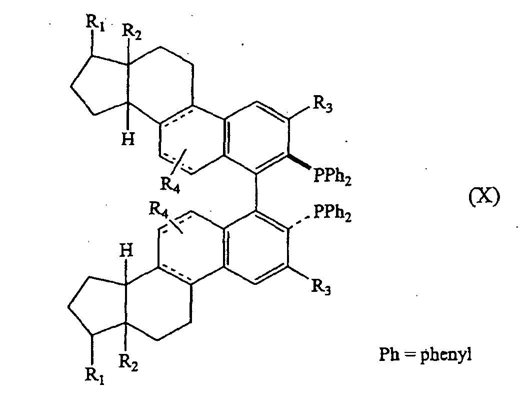
in which R₁ represents a hydrogen, a fluoride, an alkyl or acyl group, or a group of formula XR₅ in which X is oxygen or sulfur and R₅ is a hydrogen or an alkyl or aryl group;
R₂ represents a hydrogen or an alkyl group, with the stereochemistry of the C-₁₃, C-₁₄ and C-₁₇ being α or β;
R₃ represents a hydrogen, a fluoride, an alkyl group, an aryl or trialkylsilyl group or a group of formula XR₆ in which X is as defined above and R₆ represents an hydrogen or an alkyl, aryl or trifluoromethylsulfonyl group ;
R₄ is a substituent in position 6 or 7 of the steroide and represents a hydrogen, a fluoride, an alkyl or aryl group or a group of formula YR₇ in which Y represents an oxygen or sulfur atom or a trialkylsilyl group and R₇ represents a hydrogen or an alkyl, aryl or trifluoromethylsulfonyl group; the B cycle of the steroide being saturated or carrying two double bonds.

2. Process according to claim 1, **characterized in that** there is used a ligand of the general formula 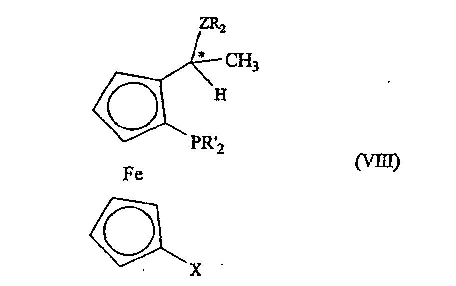 in which the carbon atom marked by the asterisk is an asymmetric carbon atom, X is a hydrogen or a bridging group bonded to a polymer as defined in claim 1, Z is a phosphorous atom, and R and R' are, independently from each other, a linear or branched alkyl group from C₁ to C₄, a cyclohexyl group or a phenyl group non-substituted or substituted by 1 to 3 alkyl groups from C₁ to C₄, which alkyl groups may be partially or entirely fluorinated;
or wherein Z is a nitrogen atom and R and R' are, independently from each other, a linear or branched alkyl group from C₁ to C₄, a cyclohexyl group, or a phenyl group non-substituted or substituted by 1 to 3 alkyl groups from C₁ to C₄.

3. Process according to claim 1 or 2, **characterized in that** there is used a ligand of formula (VIII) in which Z is a phosphorous atom, R = tert-butyl and R' = phenyl; or R = R' = cyclohexyl ; or R ≠ R' = cyclohexyl and phenyl ; or R = tert-butyl and R' = p-CF₃-phenyl ; or R = cyclohexyl, R' = p-CF₃ phenyl ; or R = cyclohexyl, R' = p-tolyl ; or Z is a nitrogen atom and R = methyl and R' = phenyl.

4. Process according to any one of claims 1 to 3, **characterized in that** X is a hydrogen atom.

5. Process according to any one of claims 1 to 3, **characterized in that** X is a group of formula (A)ₙ-B-P in which
a) n = 0 and
B is a group of formula 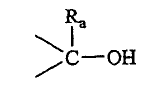 in which Rₐ is a hydrogen atom or an alkyl group from C₁ to C₁₂ ; or
b) n = 1 and
A is a group of formula -[Si(R₁₄R₁₅)]ₘR₁₆-, in which m is 0 or 1, the groups R₁₄ and R₁₅ can be identical or different and represent independently from each other an alkyl group from C₁ to C₁₂, a cycloalkyl groups from C₃ to C₇, a benzyl group or a phenyl group, or, taken together, represent an alkylene group from C₅ to C₁₂, and R₁₆ represents a phenylene group or an alkenyl group from C₁ to C₁₂, non-substituted or substituted by one or more halide atoms or hydroxy groups, and
B is a group -N(R₁₇)-C(O)-N(H)-(CH₂)ₒ-Si(R₁₈)₃₋ₚ-(O)ₚ- or a group -O-C(O)-N(H)(CH₂)ₒ-Si(R₁₈)₃₋ₚ-(O)ₚ-, in which R₁₇ represents a hydrogen atom or an alkyl group from C₁ to C₁₂, o is an integer from 1 to 12, R₁₈ is an alkyl group from C₁ to C₄ or an alkoxy group from C₁ to C₁₂ and p is an integer from 1 to 3, and
P is silica; or
c) n = 1 and
A is a group -[Si(R₁₄R₁₅)]ₘR₁₆-, in which m is 0 or 1, the groups R₁₄ and R₁₅ can be identical or different and represent independently from each other an alkyl group from C₁ to C₁₂, a cycloalkyl group from C₃ to C₇, a benzyl group, a phenyl group, or, taken together, represent an alkylene group from C₅ to C₁₂, and R₁₆ represents a phenylene group or an alkenyl group from C₁ to C₁₂, non-substituted or substituted by one or more halide atoms or hydroxy groups, and
B is selected amongst
i) the groups -N(R₁₇)-C(O)-, N(R₁₇)-C(O)-N(H)-R₁₉-N(H)-C(O)- or -O-C(O)-N(H)-R₁₉-N(H)-C(O)-, in which R₁₇ is as defined above, R₁₉ is a bridging group derived from a diisocyanate selected from the group consisting of substituted and unsubstituted C₂ to C₂₀alkylene groups, and substituted and unsubstituted phenylene, naphthylene and biphenylene groups,
ii) the groups susceptible of reacting, by polymerisation or co-polymerisation, with the monomers forming the polymer P, and
iii) the groups susceptible of reacting with the polymer P or with functional groups present on said polymer P, and
P is an organic polymer, non-functionalized or functionalized, selected from the group consisting of polyolefines, polyacrylates, polyisoprenes, polybutadienes, polystyrenes, polyphenylenes, polyvinylchloride, polyvinylidene chloride, polyallyl polymers, polyurethanes, polyethers, polyesters and polyamides.

6. Process according to claim 1, **characterized in that** there is used a ligand of formula (XI)

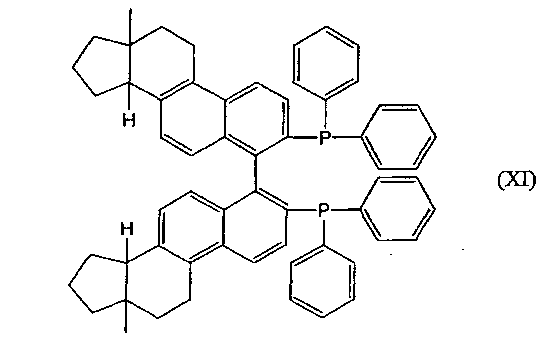

7. Process according to any one of claims 1 to 6, **characterized in that** there is used an active Rh complex of the formula [RhL∗(ene)₂]⁺X⁻, [RhL∗(diene)]⁺X⁻; [RbL∗]⁺X⁻ or [RhL∗₂]⁺X⁻, in which L∗ is a chiral phosphorous ligand as defined in any one of claims 1 to 7, "ene" is an olefin such as ethylene, propylene or butene, "diene" is a diene such as 1,3-butadiene, 1,3-pentadiene, cyclopentadiene, 1,5-hexadiene, 1,4-cyclohexadiene, 1,4- or 1,5-heptadiene, 1,4- or 1,5-cyclcoheptadiene, 1,4- or 1,5-octadiene, 1,4- or 1,5-cyclooctadiene, or norbornadiene, and X⁻ stands for an anion such as halides, ClO₄-, BF₄-, B(C₆H₅)₄-, PF₆-, PCl₆-, CH₃COO-, CF₃COO-, SbF₆-, AsF₆-, CH₃SO₃-, FSO₃- and CF₃SO₃-.

8. Process according to claim 7, **characterized in that** the diene is norbornadiene or 1,5-cyclooctadiene and the anion X- is CF₃SO₃-.

9. Process according to any one of claims 1 to 8, **characterized in that** said allyl amine is geranyldiethylamine, neryldiethylamine, cyclohexylgeranylamine, methylcyclohexylgeranylamine or (E)- or (Z)-N,N-diethyl-7-hydroxy-3,7-dimethyl-2-octenylamine.

10. Process according to any one of claims 1 to 9, **characterized in that** the active complex is used in a concentration of from about 0.05 mole% to about 20 mole% with respect to the substrate.

11. Process according to claim 10, **characterized in that** the active complex is used in a concentration of from about 0.1 to about 5 mole% with respect to the substrate.

12. Process according to any one of claims 1 to 11, **characterized in that** the reaction is carried out at a temperature of from about 0°C to about 150°C.

13. Process according to claim 12, **characterized in that** the reaction is carried out at a temperature of from about 40° to 110°C.

14. Process according to any one of claims 1 to 13, **characterized in that** the solvent used is tetrahydrofurane.

## Patentansprüche

1. Verfahren zur stereospezifischen Isomerisierung von prochiralen Allylsystemen der Formel 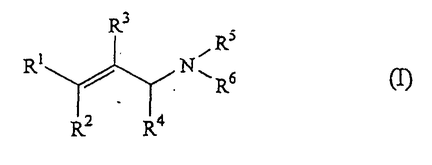 worin R¹ ≠ R² und jedes eine Alkyl- oder Alkenylgruppe mit 1 bis 12 Kohlenstoffatomen oder eine gegebenenfalls mit einer Hydroxygruppe substituierte Arylgruppe darstellt, R³ und R⁴ unabhängig voneinander Wasserstoff, eine C₁-C₁₂-Alkyl- oder -Alkenylgruppe oder eine Arylgruppe darstellen, R⁵ Wasserstoff oder eine Alkyl- oder Cycloalkylgruppe mit 1 bis
8 Kohlenstoffatomen ist, R⁶ eine Alkyl- oder Cycloalkylgruppe mit 1 bis
8 Kohlenstoffatomen ist, oder R⁵ und R⁶ zusammen mit dem Stickstoff einen Ring mit 5 oder 6 Atomen oder auch einen Sauerstoff enthaltenden Ring mit 6 Atomen bilden,
zu Enaminen der Formel 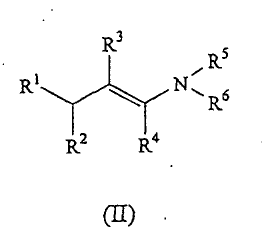 worin die Symbole R¹-R⁶ die oben angegebene Bedeutung haben, oder zu Iminen der Formel 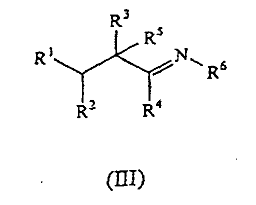 worin die Symbole R¹-R⁴ und R⁶ die oben angegebene Bedeutung haben und R⁵ Wasserstoff ist, mittels Rh-, Ir- oder Ru-Katalysatoren, die wenigstens einen chiralen phosphorhaltigen Liganden tragen, wobei das Verfahren **dadurch gekennzeichnet ist, dass** der phosphorhaltige Ligand ein Ligand der Formel (V) ist 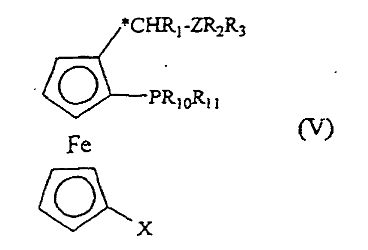 worin das mit einem Stern markierte Kohlenstoffatom ein asymmetrisches. Kohlenstoffatom darstellt, X ein Wasserstoffatom oder eine an ein Polymer gebundene verbrückende Gruppe der Formel (A)ₙ-B-P ist, worin
n = 0 oder 1,
A eine passende Vorläufergruppe ist, die wenigstens ein Atom enthält, das ausgewählt ist aus Sauerstoff, Schwefel, Silicium, Stickstoff und Kohlenstoff,
B eine Gruppe ist, die das Polymer P und den Cyclopentadienylrest verknüpft, und
P ein organisches oder anorganisches Polymer ist, und
A. Z ein Phosphoratom darstellt; R₁ eine C₁-C₈-Alkylgruppe, eine Phenylgruppe oder eine mit 1 bis 3 C₁-C₄-Alkyl- oder -Alkoxygruppen substituierte Phenylgruppe darstellt; R₂, R₃, R₁₀ und R₁₁ unabhängig voneinander eine C₁-C₁₂-Alkylgruppe, eine gegebenenfalls mit einer C₁-C₄-Alkyl- oder -Alkoxygruppe substituierte C₅-C₁₂-Cycloalkylgruppe oder eine gegebenenfalls mit 1 bis 3 Gruppen, ausgewählt aus C₁-C₄-Alkyl- oder -Alkoxygruppen, -SiR₄R₅R₆, Halogen, -SO₃M, -CO₂M, -PO₃M, -NR₇R₈ oder -[⁺NR₇R₈R₉]Y⁻, substituierte Phenylgruppe bedeuten; oder R₂, R₃, R₁₀ und R₁₁ unabhängig voneinander einen Rest der Formel 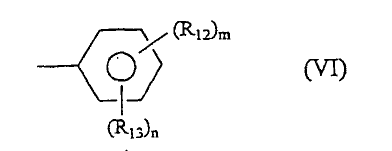 darstellen, worin R₁₂ eine ganz oder teilweise mit Fluor substituierte C₁-C₅-Alkylgruppe darstellt;
R₁₃ eine C₁-C₄-Alkyl- oder -Alkoxygruppe, -SiR₄R₅R₆, Halogen, -SO₃M, -CO₂M, -PO₃M, -NR₇R₈ oder -[⁺NR₇R₈R₉]Y⁻ darstellt;
m eine Zahl von 1 bis 3 ist; n 0 oder eine Zahl von 1 bis 4 ist, wobei die Summe aus m + n 1 bis 5 ist;
wobei R₄, R₅ und R₆ oben unabhängig voneinander eine C₁-C₁₂-Alkylgruppe oder eine Phenylgruppe bedeuten; R₇ und R₈ getrennt jeweils ein Wasserstoffatom, eine C₁-C₁₂-Alkylgruppe oder eine Phenylgruppe bedeuten oder zusammen eine Tetramethylen-, Pentamethylen oder 3-Oxa-1,5-pentylengruppe darstellen; und R₉ Wasserstoff oder eine C₁-C₄-Alkylgruppe darstellt;
M H oder ein Alkalimetall bedeutet und Y⁻ das Anion einer einbasigen Säure bedeutet;
oder
B. Z Stickstoff darstellt; R₁ eine C₁-C₈-Alkylgruppe, eine Phenylgruppe oder eine mit 1 bis 3 C₁-C₄-Alkyl- oder -Alkoxygruppen substituierte Phenylgruppe darstellt, R₂ und R₃ unabhängig voneinander eine C₁-C₁₂-Alkylgruppe, eine C₅-C₁₂-Cycloalkylgruppe oder eine Phenylgruppe, die mit 1 bis 3 C₁-C₄ Alkylgruppen substituiert sein kann, darstellen; oder R₂ und R₃ zusammen mit dem Stickstoff einen Ring mit 5 bis 12 Atomen bilden, der weitere Sauerstoff- und Stickstoffatome enthalten kann; und R₁₀ und R₁₁ jeweils eine Phenylgruppe darstellen; oder
der Ligand die allgemeine Formel hat 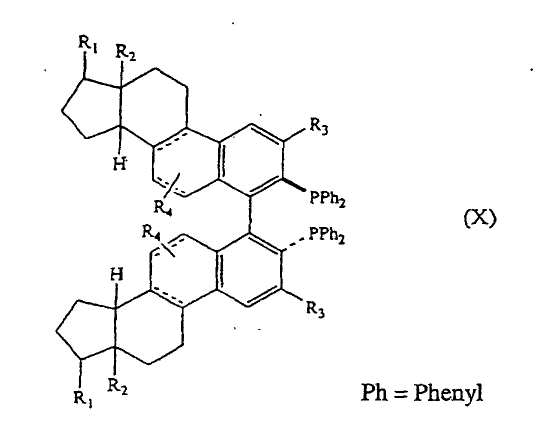
worin R₁ Wasserstoff, Fluorid, eine Alkyl- oder Acylgruppe, oder eine Gruppe der Formel XR₅ darstellt, in welcher X ein Sauerstoff- oder Schwefelatom darstellt und R₅ Wasserstoff oder eine Alkyl- oder Arylgruppe ist;
R₂ Wasserstoff oder eine Alkylgruppe darstellt, wobei die Stereochemie der Atome C-₁₃, C-₁₄ und C-₁₇ α oder β sein kann;
R₃ Wasserstoff, Fluorid, eine Alkyl-, Aryl oder Trialkylsilylgruppe oder eine Gruppe der Formel XR₆ darstellt, in welcher X die oben angegebene Bedeutung hat und R₆ Wasserstoff oder eine Alkyl-, Aryl- oder Trifluormethylsulfonylgruppe darstellt;
R₄ ein Substituent in Stellung 6 oder 7 des Steroids ist und Wasserstoff, Fluorid, eine Alkyl- oder Arylgruppe oder eine Gruppe der Formel YR₇ darstellt, in welcher Y ein Sauerstoff- oder Schwefelatom oder eine Trialkylsilylgruppe darstellt und R₇ Wasserstoff oder eine Alkyl-, Aryl- oder Trifluormethylsulfonylgruppe darstellt; wobei der Ring B des Steroids gesättigt ist oder Doppelbindungen trägt.

2. Verfahren nach Anspruch 1, **dadurch gekennzeichnet, dass** man einen Liganden der allgemeinen Formel 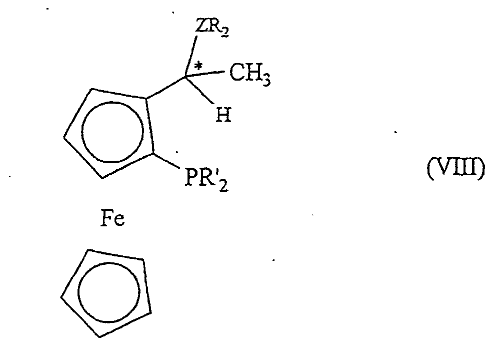 verwendet, worin das mit einem Stern markierte Kohlenstoffatom ein asymmetrisches Kohlenstoffatom ist, X Wasserstoff oder eine wie in Anspruch 1 definierte an ein Polymer gebundene verbrückende Gruppe ist, Z ein Phosphoratom ist und R und R' unabhängig voneinander eine C₁-C₄-Alkylgruppe, linear oder verzweigt, eine Cyclohexylgruppe oder eine Phenylgruppe bedeuten, die unsubstituiert oder mit 1 bis 3 C₁-C₄-Alkylgruppen substituiert ist, wobei letztere ganz oder teilweise fluoriert sein können;
oder worin Z ein Stickstoffatom ist, R und R' unabhängig voneinander eine C₁-C₄-Alkylgruppe, linear oder verzweigt, eine Cyclohexylgruppe oder eine Phenylgruppe bedeuten, die unsubstituiert oder mit 1 bis 3 C₁-C₄-Alkylgruppen substituiert ist.

3. Verfahren nach Anspruch 1 oder 2, **dadurch gekennzeichnet, dass** man einen Liganden der Formel VIII verwendet, worin Z ein Phosphoratom ist, R=tert.-Butyl und R'=Phenyl; oder R=R'=Cyclohexyl; oder R≠R'=Cyclohexyl oder Phenyl; oder R=tert.-Butyl und R'=p-CF₃-Phenyl; oder R=Cyclohexyl und R'=p-CF₃-Phenyl; oder R=Cyclohexyl und R'=p-Tolyl; oder Z ein Stickstoffatom ist und R=Methyl und R'=Phenyl.

4. Verfahren nach einem der Ansprüche 1 bis 3, **dadurch gekennzeichnet, dass** X ein Wasserstoffatom ist.

5. Verfahren nach einem der Ansprüche 1 bis 3, **dadurch gekennzeichnet, dass** X eine Gruppe (A)ₙ-B-P ist, worin
a) n = 0 und
B ein Rest der Formel 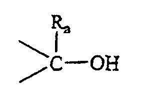 ist, worin Rₐ ein Wasserstoffatom oder eine C₁-C₁₂-Alkylgruppe ist; oder
b) n = 1 und
A eine Gruppe -[Si(R₁₄R₁₅)]ₘR₁₆- ist, in welcher m 0 oder 1 ist, die Reste R₁₄ und R₁₅ gleich oder verschieden sein können und unabhängig voneinander eine C₁-C₁₂-Alkylgruppe, eine C₃-C₇-Cycloalkylgruppe, eine Benzylgruppe, eine Phenylgruppe darstellen oder zusammen eine C₅-C₁₂-Alkylengruppe darstellen, und wobei R₁₆ eine Phenylengruppe oder eine C₁-C₁₂-Alkylengruppe, unsubstituiert oder mit ein oder mehreren Halogenatomen oder OH-Gruppen substituiert, darstellt, und
B eine Gruppe
- N(R₁₇)-C(O)-N(H)-(CH₂)ₒ-Si(R₁₈)₃₋ₚ-(O)ₚ- oder eine Gruppe - O-C(O)-N(H)(CH₂)ₒ-Si(R₁₈)₃₋ₚ-(O)ₚ- ist, in welchen R₁₇ ein Wasserstoffatom oder eine C₁-C₁₂-Alkylgruppe darstellt, o eine ganze Zahl von 1 bis 12 ist, R₁₈ eine C₁-C₄-Alkylgruppe oder eine C₁-C₁₂-Alkoxygruppe ist, und p eine ganze Zahl von 1 bis 3 ist, und
P Kieselsäure ist; oder
c) n = 1 und
A eine Gruppe -[Si(R₁₄R₁₅)]ₘR₁₆- ist, in welcher m 0 oder 1 ist, die Reste R₁₄ und R₁₅ gleich oder verschieden sein können und unabhängig voneinander eine C₁-C₁₂ Alkylgruppe, eine C₃-C₇-Cycloalkylgruppe, eine Benzylgruppe, eine Phenylgruppe darstellen oder zusammen eine C₅-C₁₂-Alkylengruppe darstellen, und wobei R₁₆ eine Phenylengruppe oder eine C₁-C₁₂-Alkylengruppe, unsubstituiert oder mit ein oder mehreren Halogenatomen oder OH-Gruppen substituiert, darstellt, und
B ausgewählt ist aus
i) den Gruppen -N(R₁₇)-C(O)-, N(R₁₇)-C(O)-N(H)-R₁₉-N(H)-C(O)- oder - O-C(O)-N(H)-R₁₉-N(H)-C(O)-, in welchen R₁₇ die oben angegebene Bedeutung hat, R₁₉ eine verbrückende Gruppe ist, die sich von einem Diisocyanat ableitet, ausgewählt aus substituierten oder unsubstituierten C₂-C₂₀-Alkylengruppen, substituierten oder unsubstituierten Phenylen-, Naphthylen- und Biphenylengruppen,
ii) Gruppen, die durch Polymerisation oder Copolymerisation mit dem Monomer, das das Polymer P bildet, reagieren können, und
iii) Gruppen, die mit dem Polymer P oder funktionellen Gruppen auf dem Polymer P reagieren können, und
P ein funktionalisiertes oder nicht funktionalisiertes organisches Polymer ist und ausgewählt ist aus Polyolefinen, Polyacrylaten, Polyisoprenen, Polybutadienen, Polystyrolen, Polyphenylenen, Polyvinylchlorid, Polyvinylidenchlorid, Polyallylpolymeren, Polyurethanen, Polyethern, Polyestern und Polyamiden.

6. Verfahren nach Anspruch 1, **dadurch gekennzeichnet, dass** man einen Liganden der Formel (XI) verwendet.

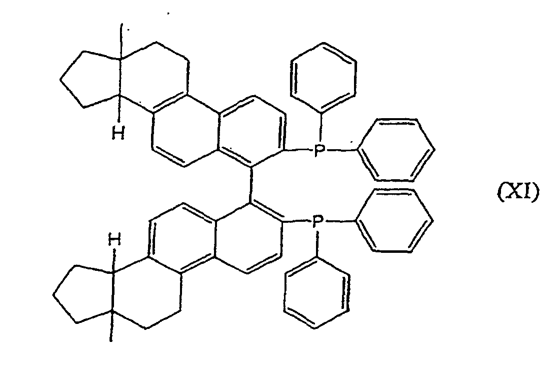

7. Verfahren nach einem der Ansprüche 1 bis 6, **dadurch gekennzeichnet, dass** man einen aktiven Rh-Komplex der Formel [RhL∗(en)₂]⁺X⁻; [RhL∗dien] ⁺X⁻; [RhL∗]⁺X⁻; oder [RhL∗₂] ⁺X⁻; verwendet, in welchen L∗ einen wie in einem der obigen Ansprüche 1 bis 7 definierten chiralen phosphorhaltigen Liganden darstellt, "en" ein Olefin wie Ethylen, Propylen oder Buten darstellt, "dien" ein Dien wie 1,3-Butadien, 1,3-Pentadien, Cyclopentadien, 1,5-Hexadien, 1,4-Cyclohexadien, 1,4- oder 1,5-Heptadien, 1,4- oder 1,5-Cycloheptadien, 1,4-oder 1,5-Octadien, 1,4- oder 1,5-Cyclooctadien, oder Norbornadien darstellt, und X⁻ ein Anion wie ein Halogenid, ClO₄⁻, BF₄⁻, B(C₆H₅)₄⁻, PF₆⁻, PCl₆⁻, CH₃COO⁻, CF₃COO⁻, SbF₆⁻, AsF₆⁻, CH₃SO₃⁻, FSO₃⁻ oder CF₃SO₃⁻ bedeutet.

8. Verfahren nach Anspruch 7, **dadurch gekennzeichnet, dass** das Dien Norbornadien oder 1,5-Cyclooctadien ist und das Anion X⁻ CF₃SO₃⁻ ist.

9. Verfahren nach einem der Ansprüche 1 bis 8, **dadurch gekennzeichnet, dass** das allylische Amin Geranyldiethylamin, Neryldiethylamin, Cyclohexylgeranylamin, Methylcyclohexylgeranylamin oder (E)- oder (Z)-N,N-Diethyl-7-hydroxy-3,7-dimethyl-2-octenylamine ist.

10. Verfahren nach einem der Ansprüche 1 bis 9, **dadurch gekennzeichnet, dass** der aktive Komplex in einer Konzentration zwischen 0,05 Mol-% und 20 Mol-%, bezogen auf das Substrat, eingesetzt wird.

11. Verfahren nach Anspruch 10, **dadurch gekennzeichnet, dass** der aktive Komplex in einer Konzentration zwischen 0,1 und 5 Mol-%, bezogen auf das Substrat, eingesetzt wird.

12. Verfahren nach einem der Ansprüche 1 bis 11, **dadurch gekennzeichnet, dass** die Reaktion in einem Temperaturbereich von 0 bis 150°C durchgeführt wird.

13. Verfahren nach Anspruch 12, **dadurch gekennzeichnet, dass** die Reaktion bei Temperaturen zwischen 40 und 110°C durchgerührt wird.

14. Verfahren nach einem der Ansprüche 1 bis 13, **dadurch gekennzeichnet, dass** das verwendete Lösungsmittel Tetrahydrofuran ist.
